(19) Europäisches Patentamt
European Patent Office
Office européen des brevets

(11) EP 4 606 900 A1

(12) EUROPEAN PATENT APPLICATION

(43) Date of publication:
**27.08.2025 Bulletin 2025/35**

(21) Application number: **24461529.0**

(22) Date of filing: **21.02.2024**

(51) International Patent Classification (IPC):
***C12N 15/85*** *(2006.01)* ***C12N 15/88*** *(2006.01)*

(52) Cooperative Patent Classification (CPC):
**C12N 15/85; A61K 48/0025; A61K 48/005; C12N 9/22; C12N 15/88;** A61K 48/0041; C12N 2310/317

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicant: **ExploRNA Therapeutics Sp. z o. o.**
**02-089 Warszawa (PL)**

(72) Inventors:
- **Golab, Jakub**
  **02-392 Warsaw (PL)**
- **Nowis, Dominika**
  **02-392 Warsaw (PL)**
- **Kowalska, Joanna**
  **02-109 Warsaw (PL)**
- **Jemielity, Jacek**
  **02-353 Warsaw (PL)**
- **Sklepkiewicz, Piotr**
  **02-389 warsaw (PL)**
- **Smietanski, Miroslaw**
  **01-864 Warsaw (PL)**
- **Sokolowska, Olga**
  **05-075 Warsaw (PL)**
- **Turowski, Pawel**
  **03-133 Warsaw (PL)**
- **Kedzierska, Hanna**
  **05-092 Lomianki (PL)**
- **Baranowski, Marek**
  **01-355 Warsaw (PL)**
- **Spiewla, Tomasz**
  **34-146 Stryszow (PL)**
- **Popielec, Agnieszka**
  **05-091 Zabki (PL)**

(74) Representative: **Eisenführ Speiser**
**Patentanwälte Rechtsanwälte PartGmbB**
**Gollierstraße 4**
**80339 München (DE)**

Remarks:
The complete document including Reference Table(s) and the Sequence Listing(s) can be downloaded from the EPO website

# (54) MODIFIED RNA FOR THE TREATMENT OF CFDNA-ASSOCIATED DISEASES

(57) The invention relates to a modified RNA with a synthetic cap structure and a modified coding region encoding for DNASE1 and/or DNASE1 L3. The invention further relates to a saRNA and/or a circular RNA, and to a vector encoding the modified RNA. The invention also relates to a pharmaceutical composition for the use of treating a disease associated with the formation of extracellular DNA, including extracellular traps released from leukocytes, such as NETs.

EP 4 606 900 A1

## Description

### Technical Field

The invention relates to a modified RNA with a synthetic cap structure and a modified coding region encoding for DNASE1 and/or DNASE1 L3. The invention further relates to a saRNA and/or a circular RNA, and to a vector encoding the modified RNA. The invention also relates to a pharmaceutical composition for the use of treating a disease associated with the formation of extracellular DNA, including extracellular traps released from leukocytes, such as NETs.

### Background of the invention

### Introduction

Cell-free DNA (cfDNA) is used to describe fragments of extracellular DNA that can be found in the plasma or extracellular environment of normal or disease-associated tissues. The blood concentrations of cfDNA vary in normal individuals, ranging from 1-50 ng/mL, but increase to over a thousand ng/mL in patients with various diseases such as cancer. Approximately 75% of cfDNA in cancer patients derives from leukocytes. At least two mechanisms contribute to the formation of cfDNA: passive release from dying cells and active secretion, mainly from myeloid cells, activated during inflammation. The latter process involves the release of genomic and/or mitochondrial DNA (mtDNA) from activated neutrophils, eosinophils, or macrophages and is a major source of cfDNA (cf. section References below, ref. 1). Neutrophil extracellular traps (NETs) consist of chromatin and mtDNA coated with enzymes, nuclear proteins, and/or antimicrobial proteins. The release of NETs from neutrophils is referred to as NETosis. NETs immobilize and neutralize pathogens, but can also damage host cells, activate platelets, and promote thrombosis. Many bacteria induce the release of NETs to avoid immune recognition and utilize cfDNA in the formation of biofilm that further impairs immune defense as well as penetration of antimicrobial drugs. The latter process contributes to developing drug (e.g. antibiotic) resistance. cfDNA and NETs play a role in the pathogenesis of many acute and chronic diseases. For example, in the context of acute respiratory distress syndrome (ARDS), uncontrolled neutrophil activation can lead to the release of NETs, composed of DNA, histones, and antimicrobial proteins (refs.1-6). These NETs contribute to the formation of fibrin clots and exacerbate tissue damage. Additionally, ARDS is marked by the accumulation of cfDNA in the lungs. This extracellular DNA, originating from damaged cells and NETs contributes to the formation of thick, viscous mucus in the airways. This viscous mucus obstructs the air passages, impairs oxygen diffusion, and exacerbates the inflammatory response, creating a challenging scenario for patients with ARDS.

Cell-free DNA (cfDNA) stimulates immune responses, leading to inflammation which can promote cancer progression. CfDNA-associated histones, acting as damage-associated molecular patterns (DAMPs), trigger toll-like receptors (TLRs), causing the release of inflammatory cytokines that further support tumor growth. Additionally, tumors induce neutrophils to release NETs, which protect tumor cells from immune attacks, hinder chemotherapy penetration, and promote thrombosis (refs. 5, 6). These findings underscore the high need to target cfDNA as a cancer treatment.

However, although, a number of attempts have been undertaken to deliver recombinant enzymes (refs. 7-11), the durability of recombinant DNASE enzymes in the human body is not sufficient to exert a lasting therapeutic effect in any therapeutically relevant setting.

In comparison to other DNASES, the therapeutic potential of DNASE1L3 is particularly poorly characterized. DNASE1L3 seems to be a regulator of self-tolerance towards DNA and DNA-associated proteins. Experimental data indicate that DNASE1L3 is involved in cleaving cfDNA derived from dead cells or actively released from myeloid cells infiltrating tumors or other diseased tissues and thus participates in regulating autoimmune responses to self-DNA, chromatin and DNA-associated proteins both in humans and in mice (refs. 12-19). Thus, although a promising target, a working therapeutic application of DNASE1IL3 is still missing.

The application of recombinant DNASES for therapeutic purposes is associated with several enormous challenges that arise from the fact that the half-life of recombinant proteins in the serum is too short to achieve a therapeutic effect after a single administration, while frequent, repeated administration may be associated with serious side effects.

In sum, there is an urgent need for an effective and gentle treatment method targeting diseases associated with cell-free DNA (cfDNA), such as cancer and inflammatory conditions, as current treatments come with severe side effects and lack long-term efficacy.

The ideal therapy should effectively address the root causes and symptoms of these diseases while minimizing adverse effects and be tailoring the therapy to individual medical needs.

Given the chronic nature of conditions like cancer and inflammatory diseases, a treatment that offers lasting relief, while minimizing adverse effects and thus improving the quality of patients' lives would be an enormous progress. This also implies a reduction in the frequency of treatments, consequently lessening the overall healthcare burden.

Thus, the present invention aims to solve the above-mentioned problems underlying the limited efficacy of recombinant DNASES against cfDNA-associated diseases by employing a novel therapeutic agent using advanced mRNA technology.

## Summary of the Invention

The above-identified objects are solved by the technical teaching as provided with the present invention.

In a first aspect there is provided a composition comprising at least one modified RNA, wherein the at least one modified RNA comprises or consists of (i) a synthetic cap structure, wherein the synthetic cap structure is selected from the group of formula (I)

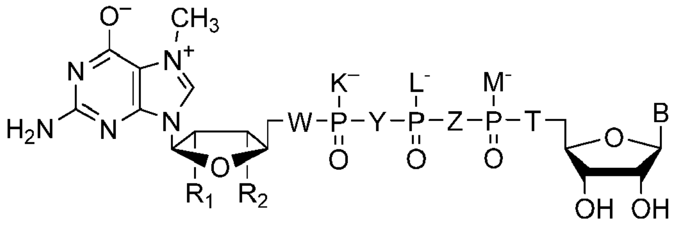

(I),

wherein B is a natural, modified, or unnatural nucleobase, W and T are independently selected from O, S, $CH_2$, $CF_2$ and $CCl_2$, Y and Z are independently selected from O, $CH_2$, $CF_2$ and $CCl_2$, K, L, and M are independently selected from O, S and BHs, and $R^1$ and $R^2$ are independently selected from the group consisting of H, OH, $OCH_3$, O-alkyl, O-alkyl derivative and O-alkylaryl, formula (II)

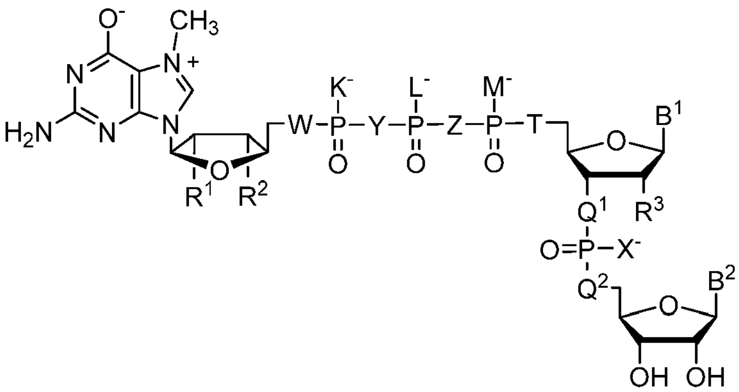

(II),

wherein $B^1$ and $B^2$ are independently natural, modified, or unnatural nucleobases, W and T are independently selected from O, S, $CH_2$, $CF_2$ and $CCl_2$, Y and Z are independently selected from O, $CH_2$, $CF_2$ and $CCl_2$, K, L, and M are independently selected from O, S and BHs, X is selected from O, S and $BH_3$, $Q^1$ and $Q^2$ are independently selected from O, S and $CH_2$, $R^1$ and $R^2$ are independently selected from the group consisting of H, OH, OCH3, O-alkyl, O-alkyl derivative and O-alkylaryl, and $R^3$ is selected from the group consisting of OH, $OCH_3$, O-alkyl, O-alkyl derivative, O-alkylaryl, F, $N_3$, $SCH_3$, $NHCH_3$ and $CH_2CH_3$, or formula III

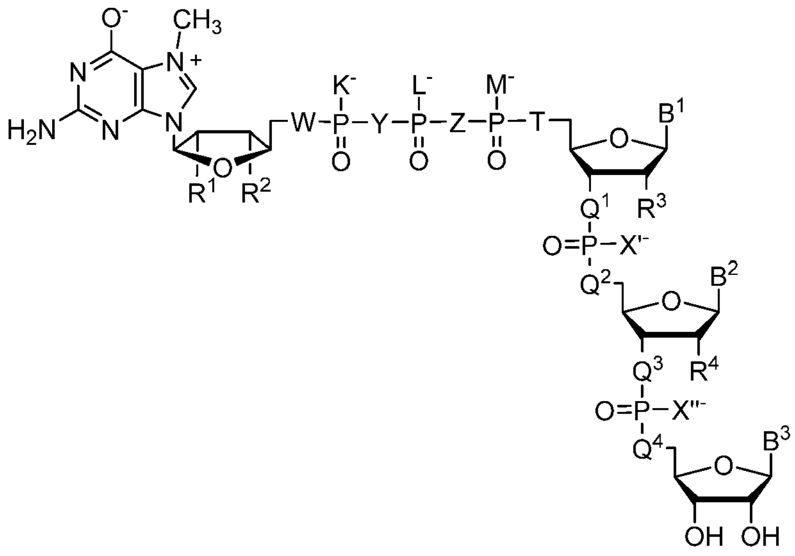

(III),

wherein $B^1$, $B^2$ and $B^3$ are independently natural, modified, or unnatural nucleobases, W and T are independently selected from O, S, $CH_2$, $CF_2$ and $CCl_2$, Y and Z are independently selected from O, $CH_2$, $CF_2$ and $CCl_2$, K, L, and M are independently selected from O, S and BHs, X' and X" are independently selected from O, S and $BH_3$, $Q^1$, $Q^2$, $Q^3$ and $Q^4$ are independently selected from O, S and $CH_2$, $R^1$ and $R^2$ are independently selected from the group consisting of H, OH, $OCH_3$, O-alkyl, O-alkyl derivative and O-alkylaryl, and $R^3$ and $R^4$ are independently selected from the group consisting of OH, $OCH_3$, O-alkyl, O-alkyl derivative, O-alkylaryl, F, $N_3$, $SCH_3$, $NHCH_3$ and $CH_2CH_3$; and (ii) at least one modified coding region encoding at least one protein, wherein the at least one protein is a DNASE1 or a DNASE1L3, or a respective homolog, ortholog or paralog thereof, preferably encoding for both DNASE1 and DNASE1L3 or a respective homolog, ortholog or paralog thereof.

In one embodiment of the present invention, there is provided a composition, wherein the DNASE1 and the DNASE1L3, or a respective homolog, ortholog or paralog thereof, is individually selected from a mammalian protein, preferably from a human protein.

In one embodiment of the present invention, there is provided a composition, wherein the DNASE1 and/or the DNASE1L3, or a respective homolog, ortholog or paralog thereof is fused to a target moiety, preferably a therapeutic antibody.

Preferably, there is provided a composition, wherein the at least one modified RNA comprises or consists of a 5'-UTR and a 3'-UTR.

Preferably, there is provided a composition, wherein the at least one modified RNA comprises or consists of a poly-A tail, wherein the poly-A tail comprises at least

(i) 100 adenines or, (ii) 120 adenines or, (iii) 150 adenines or, (iv) 250 adenines, or

(vi) a segmented poly-A tail comprising at least 150 adenines, wherein each segment is defined by the formula $A_nXA_nXA_nXA_n(XA_n)_m$, wherein A is a 5'-adenylyl moiety, X is a 5'-guanylyl or 5'-cytidylyl moiety, n is any natural number between 26 and 34, and m is 1, 2 or 3.

In further embodiments according to the present invention, there is provided a composition, wherein the modified mRNA is a self-amplifying RNA (saRNA) and or a circular RNA.

In one embodiment according to the present invention there is provided a composition, wherein the at least one modified coding region encodes for a protein or a combination of proteins defined by SEQ ID NO: 1 and 2 or a sequence having at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity to any of the SEQ ID NOs: 1 and 2, respectively.

In further embodiments according to the present invention there is provided a composition wherein said composition is provided together with at least one carrier, said carrier comprising at least one lipid nanoparticle, wherein the at least one lipid nanoparticle is selected from the group of liposomes (LPs), liposome-like nanoparticles (LLPs), solid

lipid nanoparticles (SLNs), nanostructured lipid carriers (NLCs), lipid-polymer hybrid nanoparticles (LPNs), lipoprotein particles (LPTs), nanoemulsions, cationic nanoemulsions (CNE) and exosomes.

According to second aspect of the present invention, there is provided a vector, or more than one vector, wherein the at least one vector comprises at least one nucleic acid molecule encoding the modified RNA according to the present invention.

According to a third aspect of the present invention there is provided a pharmaceutical formulation comprising the composition according to the present invention, wherein the pharmaceutical formulation additionally comprises at least one pharmaceutically acceptable carrier or excipient.

One embodiment according to the present invention relates to the pharmaceutical formulation according to the previous aspect for use in a method of treating a disease associated with the formation of extracellular DNA, preferably neutrophil extracellular traps (NETs), comprising systemically or locally administering said pharmaceutical formulation to a subject, wherein the subject is a human or a non-human mammal, optionally wherein the pharmaceutical formulation is administered in combination with an immunotherapeutic agent.

One embodiment relates to the pharmaceutical formulation for use according to the previous embodiment, wherein the disease is selected from the group consisting of cancer, deep vein thrombosis, pulmonary embolism, disseminated intravascular coagulation, inflammatory diseases, and microbial biofilm-associated diseases or medical complications.

In one aspect of the present invention there is a method of producing the at least one modified mRNA of the composition according to the present invention, the method comprising: (i) providing the at least one vector according to the second aspect, and introducing said at least one vector into at least one host cell, (ii) culturing said at least one host cell under conditions suitable for propagation and/or amplification of said at least one vector, (iii) allowing transcription from said at least one vector and obtaining said at least one modified RNA; and (iv) optionally: purifying said at least one modified RNA of step (iii); and/or (v) optionally: formulating said at least one modified RNA yielding a pharmaceutical formulation according to the third aspect, (vi) optionally: storing said at least one vector of and/or said at least one modified RNA and/or the pharmaceutical formulation.

One embodiment relates to the method according to the previous aspect further comprising a step of (vii) introducing or contacting said at least one modified RNA obtained in step (iii) into or with at least one target cell in an in vitro cellular system for transfecting said at least one target cell and/or for in vitro studying the functionality of said at least one modified RNA.

One aspect of the present invention relates to a kit comprising the at least one modified mRNA of the composition, the at least one vector and/or the pharmaceutical formulation according to the present invention, optionally wherein the kit comprises at least one further reagent and/or at least one aqueous solution, preferably a buffer.

One aspect of the present invention relates to a method for treating a disease associated with the formation of NETs, comprising administering the pharmaceutical composition according to the present invention to a subject in need thereof.

One aspect of the present invention relates to a method for treating cancer in a subject comprising administering the pharmaceutical composition according to the present invention to a subject in need thereof.

One aspect of the present invention relates to a method for treating thrombosis in a subject comprising administering the pharmaceutical composition according to the present invention to a subject in need thereof.

One aspect of the present invention relates to a method for treating ARDS in a subject comprising administering the pharmaceutical composition according to the present invention to a subject in need thereof.

One aspect of the present invention relates to a method for treating sepsis comprising administering the pharmaceutical composition according to the present invention to a subject in need thereof.

Preferably, the pharmaceutical composition according to the present invention is administered systemically and/or locally.

Preferably, the subject is a human or a mammal.

## Brief Description of the Figures

The following detailed description of the embodiments of the invention will be better understood when read in conjunction with the appended drawings. For the purpose of illustrating the invention, shown in the drawings are embodiments, which are presently exemplified. It should be understood, however, that the invention is not limited to the precise arrangement and instrumentalities of the embodiments shown in the drawings.

**Figure 1 (FIG 1):** General design of pharmaceutical compositions according to the invention.

**Figure 2 (FIG 2):** Representative analysis of mRNA integrity.

**Figure 3 (FIG 3):** Representative analysis of dsRNA content.

**Figure 4 (FIG 4):** Representative analysis of mRNA purity by HPLC.

**Figure 5 (FIG 5):** Transfection of human cells with mRNA for hDNASE1 and hDNASE1 L3 results in the generation of active enzymes efficiently degrading NETs (neutrophil extracellular traps)-derived genomic DNA. Freshly isolated human peripheral blood neutrophils were stimulated to release NETs with PMA (phorbol myristate acetate). Next, hDNASE1, hDNASE1L3, hEPO-containing medium (25 μl) or Pulmozyme® (2 U) was added to the cultures. Representative microscopic pictures of SYTOX-green-stained extracellular DNA are shown. mRNA for hEPO (protein without endonuclease activity) served as internal mRNA control.

**Figure 6 (FIG 6):** Transfection of human cells with mRNA for hDNASE1 and hDNASE1L3 results in the generation of active enzymes efficiently degrading plasmid DNA. Plasmid DNA was incubated with hDNASE1, hDNASE1L3, hEPO-containing medium or Pulmozyme® and analyzed for DNA integrity by agarose electrophoresis. mRNA for hEPO (protein without endonuclease activity) served as internal mRNA control.

**Figure 7 (FIG 7):** Administration of pharmaceutical compositions comprising DNASE-encoding mRNA and effect on tumor growth in a syngeneic mouse cancer model. The effect is significantly stronger than for DNASE administered in the form of recombinant protein (Pulmozyme®). Tumor growth inhibition (TGI) factors relative to control group up to day 21 are shown.

**Figure 8 (FIG 8):** Comparison of hDNASE1 concentrations in mouse serum after administration of DNASE-encoding mRNA or Pulmozyme® (Pulm) at indicated amounts.

The administration of DNAse in the form of mRNA yields significantly prolongated expression, which is sustained for at least 24 h, whereas after administration of Pulmozyme®, the DNAse activity falls back to background levels within 3 h. IV - intravenous administration

**Brief Description of Sequences**

| SEQ ID NO | Description |
|---|---|
| 1 | DNASE1 amino acid sequence |
| 2 | DNASE1L3 amino acid sequence |
| 3 | Representative DNASE1 nucleotide sequence |
| 4 | Representative DNASE1 L3 nucleotide sequence |
| 5 | DNase1 with UTRs variant 1 |
| 6 | DNase1 with UTRs variant 2 |
| 7 | DNase1 L3 with UTRs variant 1 |
| 8 | DNase1 L3 with UTRs variant 2 |

**Detailed Description**

To solve the problems still present in the relevant field, and to provide better and safer therapeutics, the present invention directed to mRNA-mediated DNASE delivery presents a ground-breaking advancement in medical therapeutics, particularly in the treatment of diseases associated with cell-free DNA (cfDNA), as illustrated in FIG 1. This method harnesses the capability of mRNA to ensure a prolonged elevation of DNASE levels in the bloodstream, by far outperforming the transient effects of traditional recombinant protein treatments.

Here, we discovered that administering the DNASE in the form of lipid-formulated mRNA nanoparticles (LNP) affords efficient expression of DNASES that is sustained for days and is therapeutically much more effective than administering the recombinant protein. DNASE administered in the form of lipid-formulated mRNA shows greater tumor inhibition growth factor than DNASE administered in the form of recombinant protein even if the latter is administered twice more frequently.

This sustained enzymatic activity is pivotal, especially in conditions where the durability of recombinant DNASE enzymes is insufficient for long-term therapeutic efficacy.

A key advantage of this invention lies in the facilitation of continuous in vivo production of DNASES by leveraging an mRNA:LNP-based delivery system, wherein DNASES are consistently synthesized within the patient's body, enabling a targeted and extended response against extracellular DNA. The scope of diseases that mRNA-encoded DNASES can address is impressively broad, ranging from cancer and thrombosis to a spectrum of autoimmune and inflammatory conditions, including systemic lupus erythematosus, asthma, and sepsis. This versatility underscores the potential of mRNA-mediated DNASE delivery to transform the treatment landscape for a multitude of cfDNA-dependent diseases.

Furthermore, this approach circumvents the limitations of recombinant proteins, such as dornase alfa, whose benefits are merely confined to the duration of administration. The mRNA method extends these benefits, offering a continuous therapeutic impact that can be customized to meet the unique needs of each patient.

The personalization aspect of this treatment is particularly compelling. By using the patient's specific DNASE sequence or selecting particular splice variants, therapies can be finely tuned to individual genetic profiles, enhancing both effectiveness and safety. This tailored approach is a significant stride towards precision medicine.

Moreover, the use of mRNA reduces the likelihood of immune responses and toxicity associated with repeated administrations of recombinant proteins or DNA-based delivery methods. The endogenous post-translational processing of the protein further ensures compatibility and functionality within the patient's body.

In a first aspect there is provided a composition comprising at least one modified RNA, wherein the at least one modified RNA comprises or consists of (i) a synthetic cap structure, wherein the synthetic cap structure is selected from the group of formula (I)

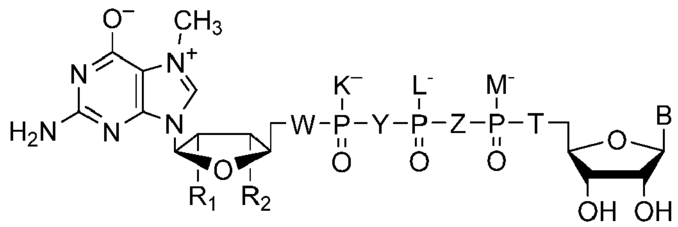

(I),

wherein B is a natural, modified, or unnatural nucleobase, W and T are independently selected from O, S, $CH_2$, $CF_2$ and $CCl_2$, Y and Z are independently selected from O, $CH_2$, $CF_2$ and $CCl_2$, K, L, and M are independently selected from O, S, and BHs, and $R^1$ and $R^2$ are independently selected from the group consisting of H, OH, $OCH_3$, O-alkyl, O-alkyl derivative and O-alkylaryl, formula (II)

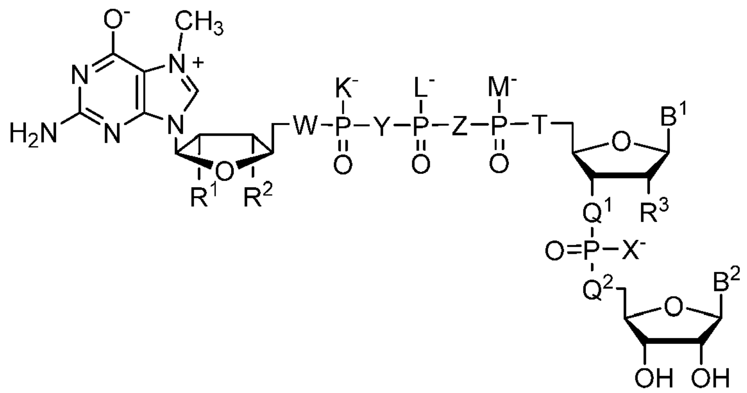

(II),

wherein $B^1$ and $B^2$ are independently natural, modified, or unnatural nucleobases, W and T are independently selected from O, S, $CH_2$, $CF_2$ and $CCl_2$, Y and Z are independently selected from O, $CH_2$, $CF_2$ and $CCl_2$, K, L, and M are independently selected from O, S and BHs, X is selected from O, S and BHs, $Q^1$ and $Q^2$ are independently selected from O, S and $CH_2$, $R^1$ and $R^2$ are independently selected from the group consisting of H, OH, OCH3, O-alkyl, O-alkyl derivative

and O-alkylaryl, and $R^3$ is selected from the group consisting of OH, $OCH_3$, O-alkyl, O-alkyl derivative, O-alkylaryl, F, $N_3$, $SCH_3$, $NHCH_3$ and $CH_2CH_3$, or formula III

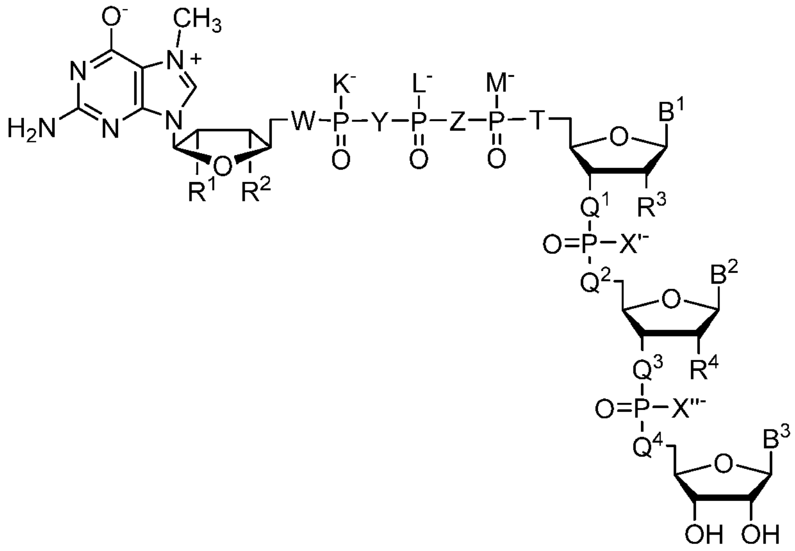

(III),

wherein $B^1$, $B^2$ and $B^3$ are independently natural, modified, or unnatural nucleobases, W and T are independently selected from O, S, $CH_2$, $CF_2$ and $CCl_2$, Y and Z are independently selected from O, $CH_2$, $CF_2$ and $CCl_2$, K, L, and M are independently selected from O, S and BHs, X' and X" are independently selected from O, S and $BH_3$, $Q^1$, $Q^2$, $Q^3$ and $Q^4$ are independently selected from O, S and $CH_2$, $R^1$ and $R^2$ are independently selected from the group consisting of H, OH, $OCH_3$, O-alkyl, O-alkyl derivative and O-alkylaryl, and $R^3$ and $R^4$ are independently selected from the group consisting of OH, $OCH_3$, O-alkyl, O-alkyl derivative, O-alkylaryl, F, $N_3$, $SCH_3$, $NHCH_3$ and $CH_2CH_3$; and (ii) at least one modified coding region encoding at least one protein, wherein the at least one protein is a DNASE1 or a DNASE1L3, or a respective homolog, ortholog or paralog thereof, preferably encoding for both DNASE1 and DNASE1L3 or a respective homolog, ortholog or paralog thereof.

In certain embodiments, there may be provided a synthetic cap structure as delineated in the first aspect above. Within these embodiments, said synthetic cap structure may include at least one diastereomer containing elements K, L, and M, where each is independently selected from S or BH3. Furthermore, each of these diastereomers can be utilized either alone or in a combined form as a diastereomeric mixture.

During the development of the invention it was found that some of the 5' cap structures confer favorable properties to mRNA that include at least one of the following features: superior quality of the in vitro transcribed RNA product, higher yield of the in vitro transcription, superior quality of the final mRNA product, augmented expression levels of DNASE in vitro and in vivo compared to mRNA capped with state-of-the-art analog (ARCA) or cap-1 structure ($m^7GpppA_mpG$):

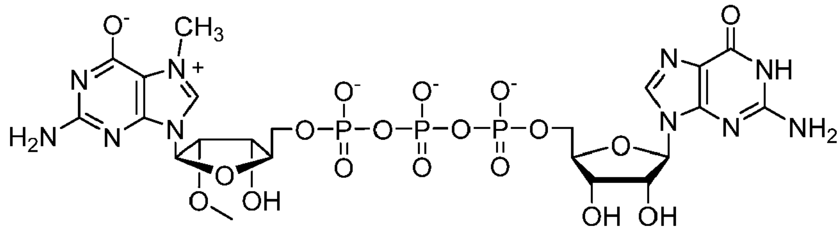

ARCA

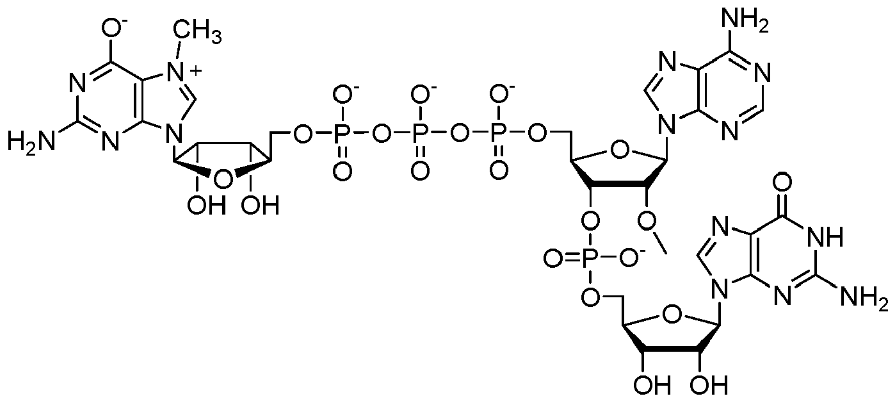

m7GpppAmpG (cap-1)

Preferably, a synthetic cap structure may be selected from the group of:

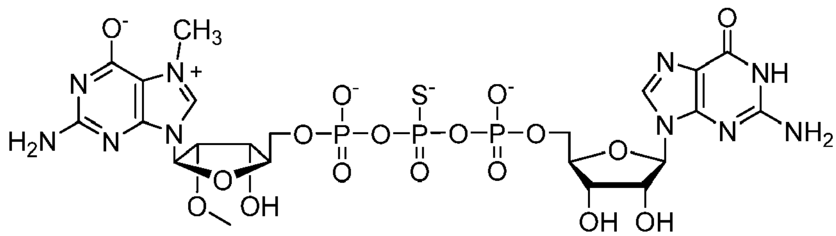

,

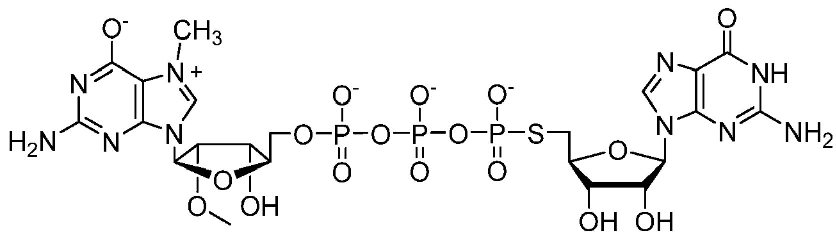

,

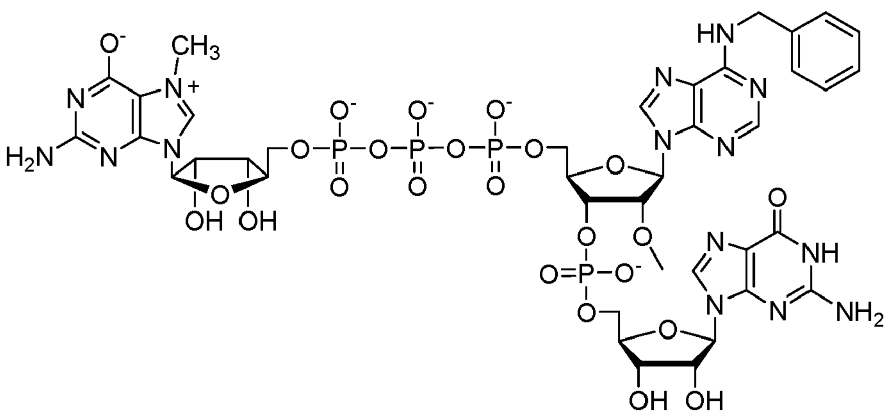

,

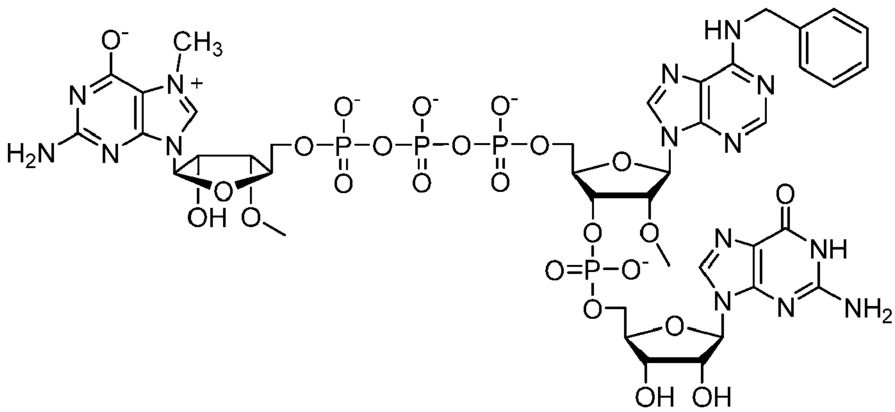

,

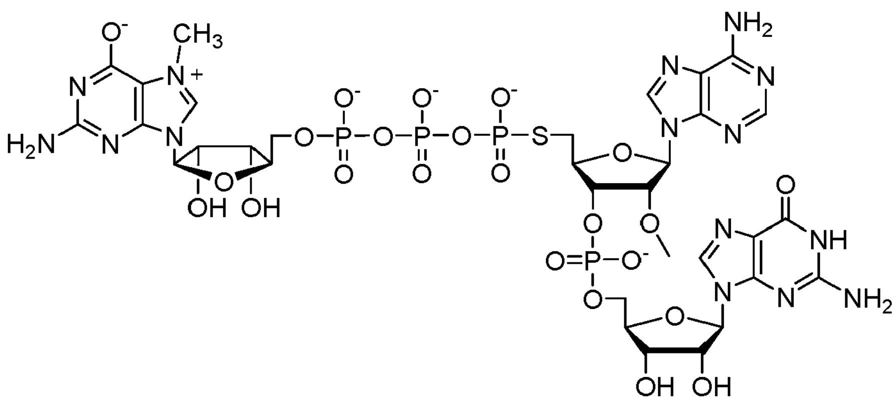

,

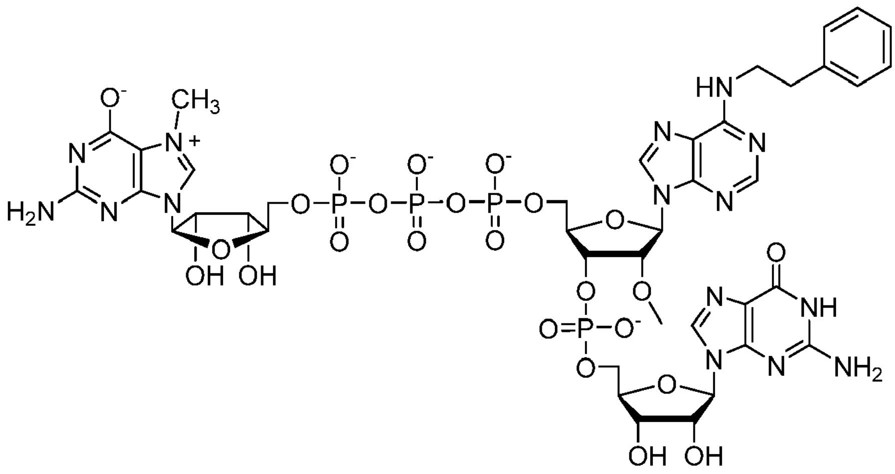

,

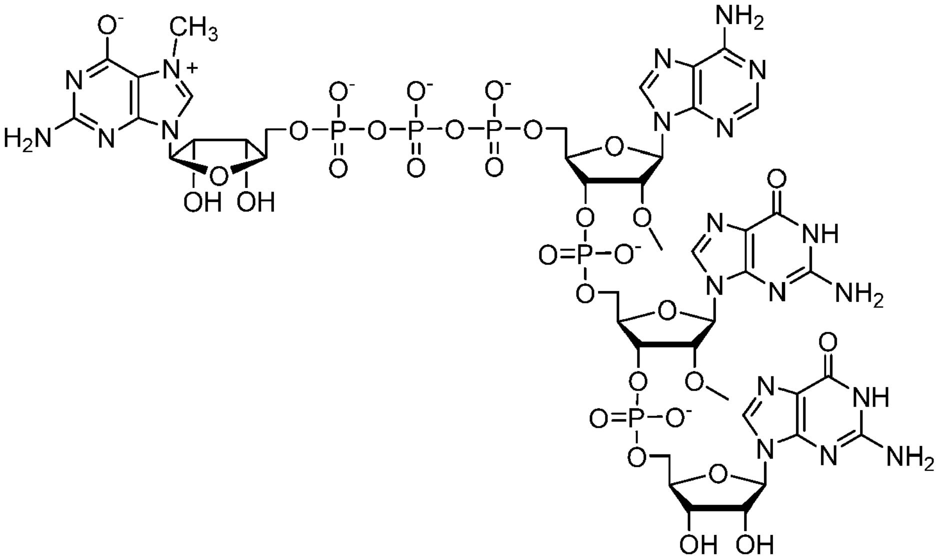

,

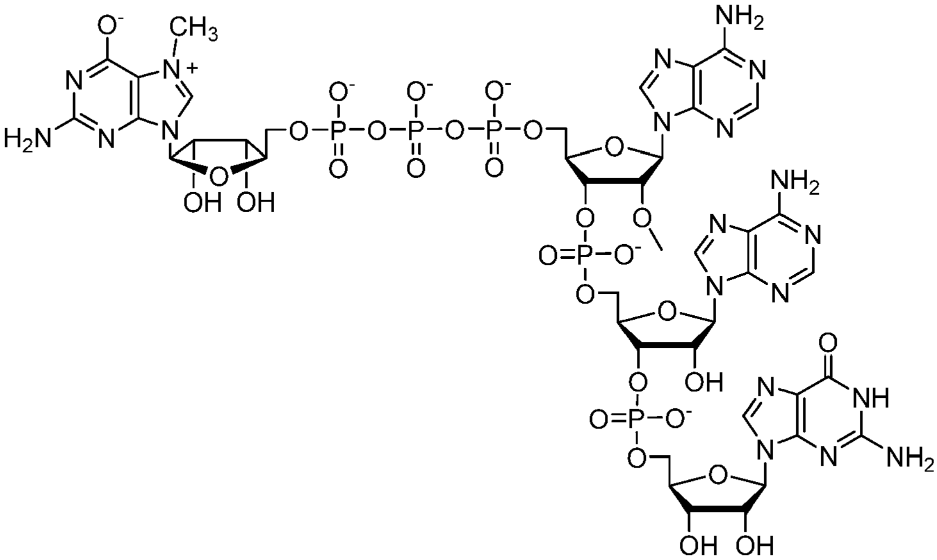

,

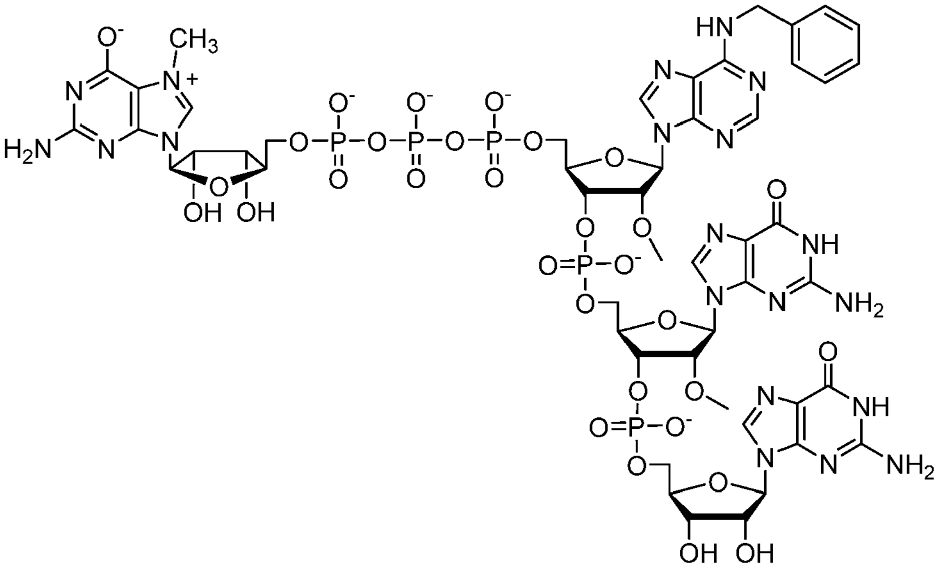

,

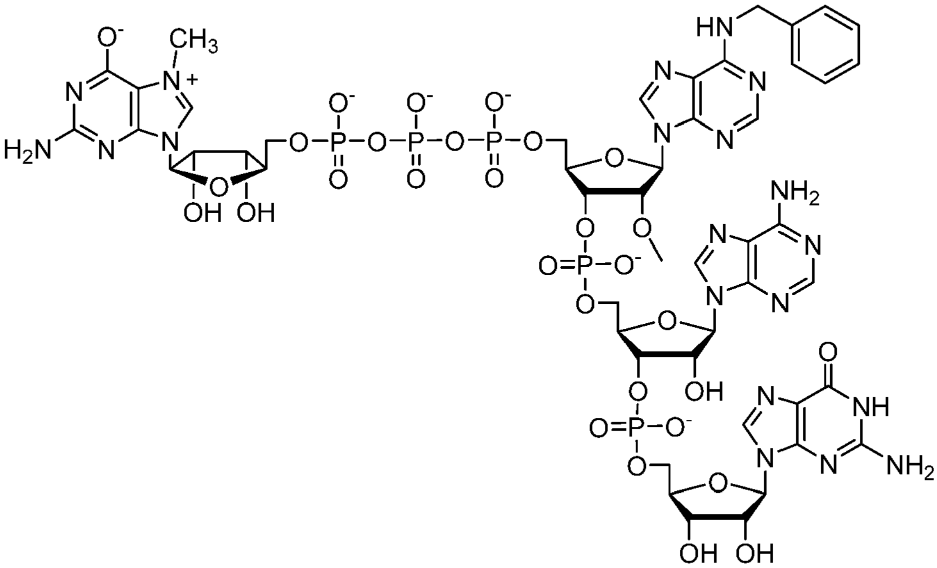

,

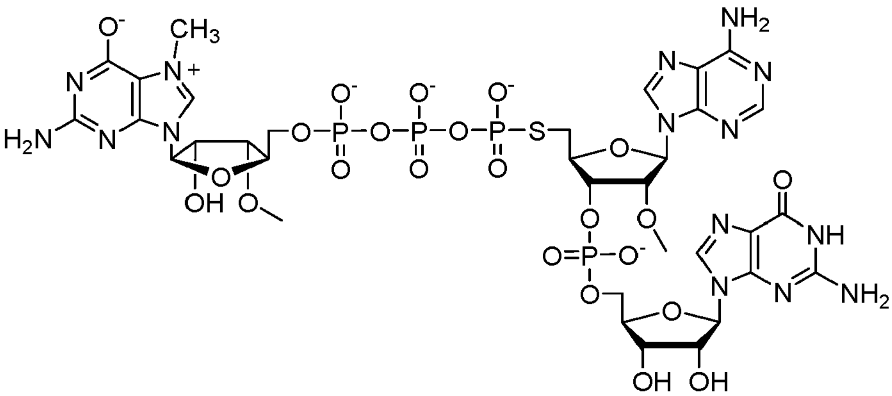

,

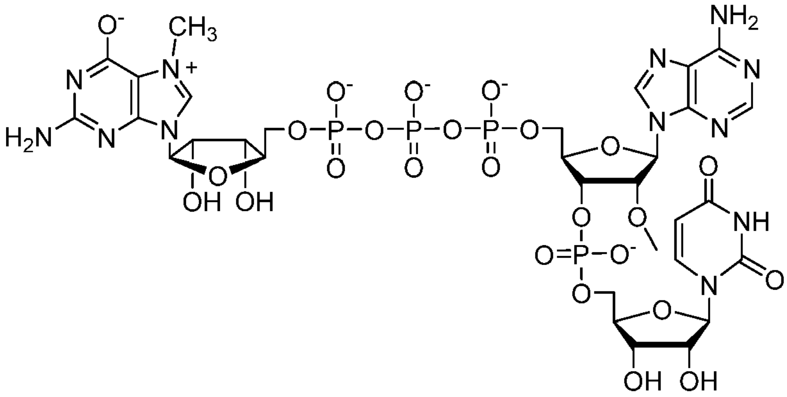

,

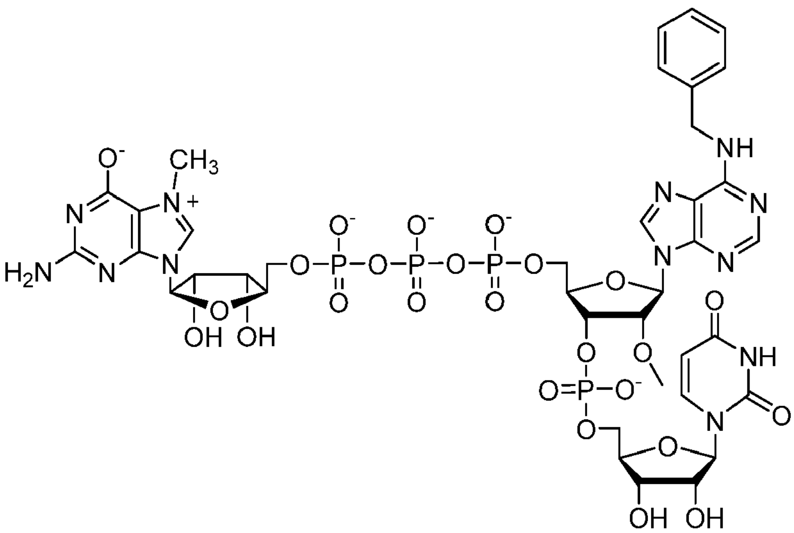

,

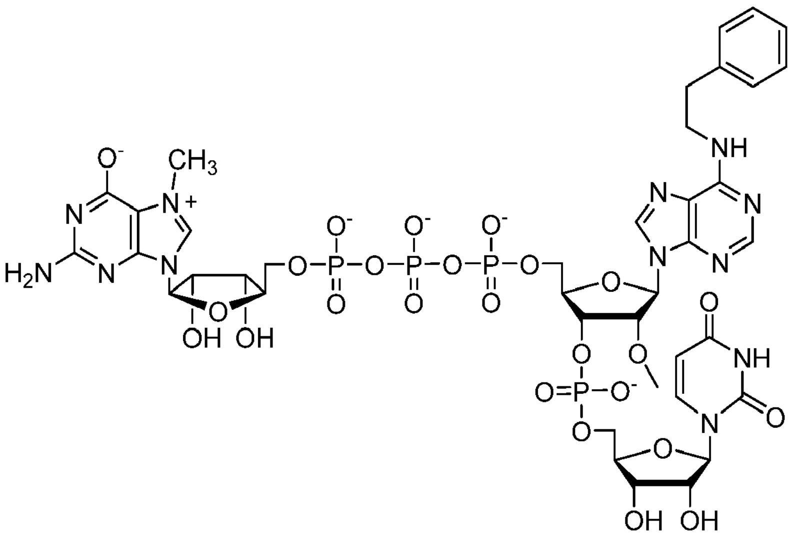

, and

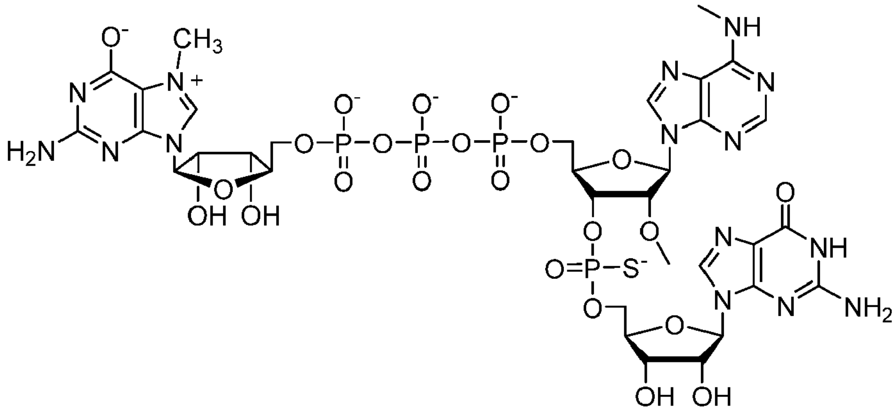

.

The present invention discloses pharmaceutical compositions comprising mRNA molecules encoding deoxyribonucleases, i.e. enzymes involved in DNA degradation: DNASE1, DNASE1L3, or a combination thereof.

The use of mRNA as a vehicle to produce DNASE in a patient's body has many advantages over the use of the recombinant protein, including longer expression and persistence of DNASE in the bloodstream as compared with administration of recombinant proteins (FIG 8) leading to less frequent dosing and increased therapeutic potency.

The use of mRNA also lowers the costs of production. It simplifies the process of modifying the sequence of RNA to deliver variants of proteins, such as patient-compatible isoforms for personalized treatment.

Using DNASE-encoding mRNA as a vehicle to achieve therapeutic proteins has no risk of insertional mutagenesis. RNA introduced into the cell cytosol undergoes rapid translation and is then degraded, in contrast to potential alternative - DNA treatment - RNA cannot integrate into the genome. Degradation of RNA allows for transient production of therapeutic protein and can be discontinued after the necessary treatment period. If DNA integrates into the genome, it can be a source of encoded protein even for a lifetime. Furthermore, RNA is less immunogenic as compared with DNA and there is no risk of inducing anti-RNA antibodies.

The administration of DNASE-encoding mRNA shows a superior effect compared to the administration of recombinant protein (commercially available DNASE1, known as Pulmozyme®. This is demonstrated by a series of constructs encoding DNASE1 and DNASE1L3 as described in Example 1. These constructs were then used to perform in vitro transcription reactions to obtain a set of capped mRNAs encoding DNASE1 and DNASE1 L3 as described in Example 2. The disclosed or very similar protocols can be used to generate similar DNASE-encoding mRNAs comprising different DNASE variants, various UTRs, various 5' terminal cap structures, and various nucleobases (i.e. either unmodified nucleobases (A,G,C, U) or modified analogs such as $\Psi$, $m^1\Psi$, $m^5C$). In parallel, mRNA constructs encoding enzymatically inactive proteins (human erythropoietin, hEPO) or ovalbumin (hOVA) were generated to serve as negative control samples. All mRNAs were formulated into lipid nanoparticles (LNPs) suitable for in vivo delivery of mRNA. The preparation, purification, and quality control of LNPs are described in Example 3. Next, we showed that DNASE produced from mRNA has an enzymatic activity in cell culture medium similar to that of recombinant DNASE 1 (Pulmozyme®) as described in Examples 4 and 5. To that end, we performed experiments using cell culture medium from HEK293 cells and either freshly isolated human peripheral blood neutrophils chemically stimulated to release NETs (Example 4) or plasmid DNA (Example 5). FIG 5 shows that 25 μl of cell culture medium collected after 24 h from transfection of HEK293T with 800 ng of mRNA encoding DNASE1 or DNASE1L3 results in comparable DNASE activity towards NETs to 2 U of Pulmozyme®.

FIG 6 shows that 10 μl of cell culture medium collected after 24 h from transfection of HEK293 cells with 800 ng of mRNA encoding DNASE1 or DNASE1L3 results in comparable DNASE activity towards plasmid DNA compared to 50 U of Pulmozyme®. The results of these experiments were used to estimate the equivalent doses of mRNA and Pulmozyme® for subsequent in vivo experiments. In vivo experiments comparing the activity of pharmaceutical formulations comprising mRNA encoding DNASE1 or DNASE1 L3 compared to recombinant DNASE protein (Pulmozyme®) were next carried out. At day 0 of the experiment, mice were inoculated with MC38 cancer cells and observed for tumor appearance and tumor growth to calculate tumor growth inhibition factors as described in Example 6. In this experiment, mice were treated at indicated days post-inoculation either with LNPs comprising 10 μg DNASE1 or 10 μg DNASE1L3 encoding mRNA or with 25 U of Pulmozyme® on days 7, 9, 11, 14, 16, 18, 21 and 23 post-inoculation. The results shown in FIG 7 demonstrate that the pharmaceutical formulations comprising DNASE1 and DNASE1L3 encoding mRNA, show more profound antitumor activity (TGI at day 21 of 54±22% and 64±18%, compared to recombinant DNASE1 (Pulmozyme®) (TGI at day 21 - 8t8%). Notably, the administration of Pulmozyme® did not show a therapeutic effect. This is likely because the administration of the pharmaceutical formulations comprising DNASE1 and DNASE1L3 encoding mRNA affords more sustained serum concentrations of DNASE protein than the administration of recombinant protein, due to prolonged

expression of protein from mRNA that partially alleviates the rapid degradation of protein in the serum.

To demonstrate this, we have measured the hDNASE levels in the blood serum of mice after intravenous (IV) administration of LNPs comprising 10 μg DNASE1-encoding mRNA or 50 μg DNASE1-encoding mRNA or recombinant protein (Pulmozyme®) as described in Example 7. We have observed a significant difference in the pharmacokinetic profiles between recombinant DNASE and LNPs comprising DNASE1-encoding mRNA (FIG 8). The DNASE concentration observed after the administration LNPs comprising 10 or 50 μg of DNASE1-encoding mRNA 3 h post-administration reached the levels comparable to the initial levels (5 min timepoint) after administration of Pulmozyme®. Similar concentrations of hDNAse were observed over the next 24 hours. The sustained hDNAse level at 24 h timepoint suggests that the expression may last even notably longer (further timepoints were not monitored). In contrast, after administration of the recombinant hDNASE (Pulmozyme®) the protein concentrations rapidly dropped reaching the background serum levels within 3 h (the background level of hDNAse1 is represented by Ctrl data bar in FIG 8). This result clearly demonstrates the advantage of using mRNA-based technology over recombinant proteins for the delivery of DNAses for therapeutic purposes.

Because DNASE1 and DNASE1 L3 have complementary activities and act together in the human body, compositions comprising mRNAs encoding both DNASE1 and DNASE1 L3 might have an enhanced biological activity compared to each enzyme alone. The DNASE1 and DNASE1 L3 proteins may be encoded by two separate RNA molecules, or a single RNA molecule encoding a fusion of DNASE1 DNASE1 L3 protein, separated by a genetically encoded protease-cleavable or non-cleavable linker.

In one embodiment, there is provided a composition, wherein the DNASE1 and the DNASE1 L3, or a respective homolog, ortholog or paralog thereof, is individually selected from a mammalian protein, preferably from a human protein.

On a sequence and structural level, the DNASE1 and DNASE1 L3 proteins are highly conserved among mammals.

The concept of homologs, orthologs and paralogs refers to the evolutionary relationships between genes or proteins. Homologs encompass a broad category, referring to any genes or proteins that originate from a common ancestral gene. This shared ancestry ties together genes across different species or within the same species, regardless of their current function or where they are found. Orthologs are genes that have evolved in different species from a common ancestral gene, separated by the event of speciation. These genes generally retain similar functions across species, despite their evolutionary divergence. On the other hand, paralogs arise within a single species and result from the duplication of a single gene in an organism's genome. Unlike orthologs, paralogs can evolve new functions or maintain functions related to the original gene. They often form gene families within a genome. This concept is thus well familiar to one skilled in the art.

In one embodiment, there is provided a composition, wherein the DNASE1 and/or the DNASE1 L3, or a respective homolog, ortholog or paralog thereof is fused to a target moiety, preferably a therapeutic antibody.

In the present invention, a target moiety is defined as a specific segment of a protein or peptide that forms a covalent bond with a target molecule, typically a receptor molecule.

A target moiety may include antibodies, which are large, Y-shaped proteins produced by the immune system, known for their high specificity and strong affinity to antigens, including receptor molecules. Therapeutic antibodies are artificially created and administered as treatments for specific diseases, such as cancer, autoimmune disorders, and infectious diseases. They are engineered for high specificity and affinity to their target antigens, offering a targeted approach to disease treatment. It also covers antibody fragments such as Fab, F(ab')2, or single-chain variable fragments (scFv). These fragments retain the antigen-binding site of antibodies but are smaller, offering different pharmacokinetic properties. Nanobodies, derived from heavy-chain-only antibodies found in camelids, represent another form of target moiety. Their smaller size compared to conventional antibodies allows for easier tissue penetration and potentially faster clearance from the body. Designed Ankyrin Repeat Proteins (DARPins) are small, protein-based binding molecules engineered for high affinity and specificity to various targets. Their compact size and stability make them versatile for numerous applications and may be fused to a protein as a target moiety. Affimers, small engineered proteins designed to bind specifically to a wide range of target molecules, offer an alternative to antibodies and other binding molecules. They have advantages in terms of size, stability, and ease of production.

Moreover, the definition of a target moiety extends beyond these examples to any molecule or molecular scaffold capable of binding to a target molecule. This includes a variety of engineered or naturally occurring proteins, peptides, or other molecular structures chosen for their specific interaction capabilities with desired targets, such as receptors or enzymes. The essential characteristic of a target moiety in this invention lies in its ability to covalently attach to and interact with a target molecule, enabling specific biological or therapeutic functions.

Preferably, there is there is provided a composition, wherein the at least one modified RNA comprises or consists of a 5'-UTR and a 3'-UTR.

5'- and 3'-UTRs (untranslated regions) flank the coding region of mRNA and influence the post-transcriptional regulation of gene expression. The 5'-UTR, positioned at the 5' end of the mRNA upstream of the start codon, is instrumental in regulating the initiation of translation. This region affects the mRNA's stability and the efficiency with which

ribosomes bind to commence protein synthesis. It may contain elements like upstream AUGs or internal ribosome entry sites, which significantly influence translational control.

On the other end, the 3'-UTR, located at the 3' end of the mRNA following the stop codon, plays a key role in determining the mRNA's stability and its localization within the cell. It includes sequences that guide the binding of regulatory proteins and small regulatory RNAs, such as microRNAs. These interactions can impact how the mRNA is degraded, its translation efficiency, and its transport within the cell. The specific sequences and structures of these UTRs are often intricately balanced to ensure the precise expression of genes in response to various internal and external cellular signals.

The UTR sequences affect the activity of the modified RNA according to the present invention. A preferred UTR sequence ensures sustainable expression of the protein encoded by the coding region.

Examples of preferred 5' and 3' UTR sequences according to the present invention are: human $\alpha$-globin 5' UTR, human $\beta$-globin 5' UTR, human $\alpha$-globin 3' UTR, human $\beta$-globin 3' UTR, human hydroxysteroid 17-beta dehydrogenase 4 gene (HSD17B4) 5' UTR, Dynein Axonemal Heavy Chain 2 (DNAH2) 5' UTR, the amino-terminal enhancer of split (AES) mRNA and the mitochondrial encoded 12S ribosomal RNA 3' UTR, human hemoglobin subunit alpha 1 gene (HBA1) 3' UTR, human hemoglobin subunit alpha 2 gene (HBA2) 3' UTR, human proteasome 20S subunit beta 3 gene (PSMB3) 3' UTR and human albumin gene (ALB) 3' UTR, or any shortened variant of any of the above, but not limited thereof. Preferably, there is provided a composition, wherein the at least one modified RNA comprises or consists of a poly-A tail, wherein the poly-A tail comprises at least (i) 100 adenines or, (ii) 120 adenines or, (iii) 150 adenines or, (iv) 250 adenines, or (vi) a segmented poly-A tail comprising at least 150 adenines, wherein each segment is defined by the formula $A_nXA_nXA_nXA_n(XA_n)_m$, wherein A is a 5'-adenylyl moiety, X is a 5'-guanylyl or 5'-cytidylyl moiety, n is any natural number between 26 and 34, and m is 1, 2 or 3.

A preferred poly-A tail sequence ensures high stability and sustained expression of the protein encoded by the coding region of the modified RNA.

The poly-A tail is a sequence of adenine nucleotides added to the 3' end of the mRNA molecule. This tail, typically comprising 50 to 250 adenine bases, serves multiple critical functions in the life cycle of mRNA. It acts as a protective buffer, shielding the mRNA from enzymatic degradation in the cytoplasm, which in turn enhances its stability and longevity within the cell. Additionally, the poly-A tail plays a pivotal role in the transport of mRNA from the nucleus to the cytoplasm, facilitating its journey to the site of protein synthesis. Beyond stability and transport, the poly-A tail significantly impacts the efficiency of translation. It aids in the recruitment of ribosomes and various translation initiation factors, making the process of translating mRNA into proteins more efficient. The length of the poly-A tail can influence the mRNA's behaviour: a longer tail is generally associated with enhanced stability and translational efficacy, whereas a shortened tail can lead to mRNA decay. This mechanism plays a crucial role in regulating gene expression, as the length of the poly-A tail can dynamically change in response to various cellular conditions, thus controlling the availability and longevity of the mRNA for protein production.

In further embodiments according to the present invention, there is provided a composition, wherein the modified mRNA is a self-amplifying RNA (saRNA) and or a circular RNA.

Self-amplifying RNA (saRNA) is a type of synthetic RNA molecule, engineered with specific genetic elements that enable it to replicate within host cells, resulting in the production of multiple copies of the RNA molecule. It comprises an inherent replication machinery, e.g. encoding an RNA-dependent RNA polymerase (RdRp). This self-replicating property allows saRNA to amplify the expression of a target antigen or protein of interest.

Circular RNA is characterized by its closed-loop, covalently linked structure, with the 3' and 5' ends joined together. The circularization may be carried out using an enzymatic, chemical, or a ribozymatic method. It is generally more stable and resistant to degradation than linear RNA. As a result, circular RNAs have a longer half-life, allowing them to persist within cells for extended periods. Another advantage is the reduced immunogenicity of circular RNA, as linear RNA molecules, when introduced into cells, are prone to trigger innate immune responses.

In one embodiment there is provided a composition, wherein the at least one modified coding region encodes for a protein or a combination of proteins defined by SEQ ID NO: 1 and 2 or a sequence having at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity to any of the SEQ ID NOs: 1 and 2, respectively.

Whenever the present disclosure relates to the percentage of identity of nucleic acid or amino acid sequences to each other these values define those values as obtained by using the EMBOSS Water Pairwise Sequence Alignments (nucleotide) programme (https://www.ebi.ac.uk/idispatcher/psa/emboss water ) nucleic acids or the EM-BOSS Water Pairwise Sequence Alignments (protein) programme (www.ebi.ac.uk/Tools/psa/emboss_water/) for amino acid sequences. Alignments or sequence comparisons as used herein refer to an alignment over the whole length of two sequences compared to each other. Those tools provided by the European Molecular Biology Laboratory (EMBL) European Bioinformatics Institute (EBI) for local sequence alignments use a modified Smith-Waterman algorithm (see https://www.ebi.ac.uk/idispatcher/psa and SMITH, T.F. 5 & WATERMAN, M.S. "Identification of common molecular subsequences" Journal of Molecular Biology, 1981 147 (1):195-197). When conducting an alignment, the default

parameters defined by the EMBL-EBI are used.

Those parameters are (i) for amino acid sequences: Matrix = BLOSUM62, gap open penalty = 10 and gap extend penalty = 0.5 or (ii) for nucleic acid sequences: Matrix = DNAfull, gap open penalty = 10 and gap extend penalty = 0.5. The skilled person is well aware of the fact that, for example, a nucleotide sequence encoding a protein can be "codon optimized" if the respective sequence is to be used in another organism in comparison to the original organism a molecule originates from.

In yet a further embodiment there is provided a composition wherein said composition is provided together with at least one carrier, said carrier comprising at least one lipid nanoparticle, wherein the at least one lipid nanoparticle is selected from the group of liposomes (LPs), liposome-like nanoparticles (LLPs), solid lipid nanoparticles (SLNs), nanostructured lipid carriers (NLCs), lipid-polymer hybrid nanoparticles (LPNs), lipoprotein particles (LPTs), nanoemulsions, cationic nanoemulsions (CNE) and exosomes.

Lipid nanoparticles (LNPs) are fundamental components in the field of mRNA delivery, playing a crucial role in the transportation and protection of mRNA molecules for a variety of biotechnological and therapeutic applications.

One of the primary functions of LNPs is to provide a protective encapsulation for mRNA. This encapsulation shields the mRNA from degradation by nucleases, ensuring the genetic information remains intact during transportation. Additionally, LNPs facilitate the efficient cellular uptake of mRNA by cells, typically through endocytosis, allowing the encapsulated mRNA to enter the cytoplasm where translation and protein synthesis occur.

LNPs contribute to the stability and bioavailability of mRNA therapeutics, safeguarding the mRNA cargo from degradation by the recipients' immune system and enhancing its pharmacokinetics. Thus, an essential attribute of LNPs is their minimal immunogenicity when properly designed, reducing the risk of undesirable immune responses against the delivery system itself. Furthermore, they can be engineered for tissue-specific targeting, enabling the targeting of specific cell types or organs for therapeutic purposes.

In one embodiment of the present invention, the carrier facilitates delivery of the composition to at least one organ selected from the group liver, lung and kidney.

Preferably, the carrier comprises at least one of the lipids listed in Table 1.

**Table 1**

| Abbrev. | Formal name | Structure |
|---|---|---|
| DOTAP | 1,2-Dioleoyl-3-trimethylammonium propane | 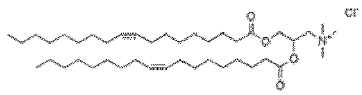 |
| DOTMA | 1,2-d i-O-octadecenyl-3-trimethylammonium propane | 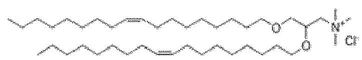 |
| 16:0 DAP | 1,2-dipalmitoyl-3-trimethylammonium-propane | 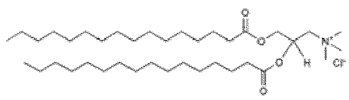 |
| 18:0 DAP | 1,2-distearoyl-3-dimethylammonium-propane | 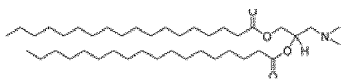 |
| DODAC | Dimethyldioleylammonium chloride | 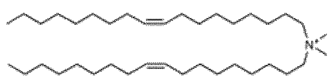 |
| ALC-0315 | [(4-Hydroxybutyl)azanediyl]di(he xane-6,1-diyl) bis(2-hexyldecanoate) | 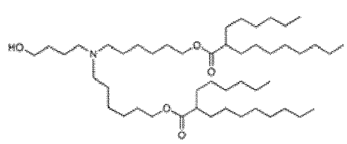 |
| SM-102 | Heptadecan-9-yl 8-((2-hydroxyethyl)(6-oxo-6-(undecyloxy)hexyl)amino)oct anoate | 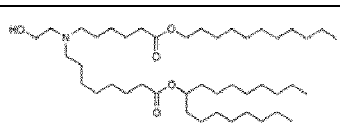 |
| DLin-MC3-DMA | (6Z,9Z,28Z,31Z)-Heptatriaconta-6,9,28,31-tetraen-19-yl 4-(dimethylamino)butanoate | 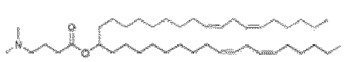 |

(continued)

| Abbrev. | Formal name | Structure |
|---|---|---|
| DLin-DMA | N,N-dimethyl-2,3-bis[(9Z,12Z)-9,12-octadecadien-1-yloxy]-1-propanamine | 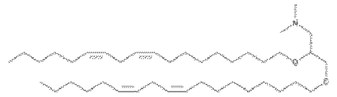 |
| DLin-KC2-DMA | N,N-dimethyl-2,2-di-(9Z,12Z)-9,12-octadecadien-1-yl-1,3-dioxolane-4-ethanamine | 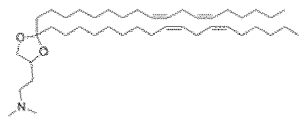 |
| C14-4 | 1,1'-[[2-[2-[4-[2-[[2-[2-[bis(2-hydroxytetradecyl)amino] etho xy]ethyl](2-hydroxytetradecyl)amino]ethy l]-1-piperazinyl]ethoxy]ethyl]imino jbis-2-tetradecanol | 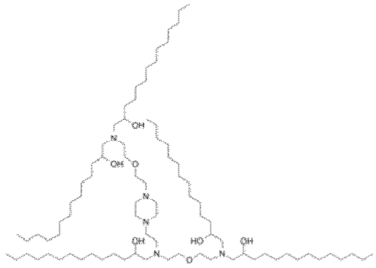 |
| C12-200 | 1,1'-[[2-[4-[2-[[2-[bis(2-hydroxydodecyl)amino]ethyl]( 2-hydroxydodecyl)amino]ethyl]-1-piperazinyl]ethyl]imino] bis-2-dodecanol | 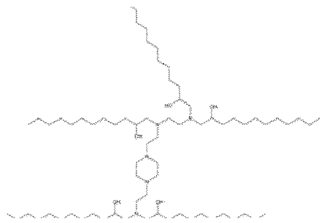 |
| 306-Oi10 | tetrakis(8-methylnonyl) 3,3',3",3‴-(((methylazanediyl) bis(propa ne-3,1-diyl))bis(azanetriyl))tetrapropi onate | 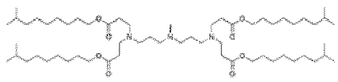 |
| 306-O12B | tetrakis(2-(octyldisulfaneyl)ethyl) 3,3',3",3‴-(((methylazanediyl)bis(propa ne-3,1-diyl))bis(azanetriyl))tetrapropi onate | 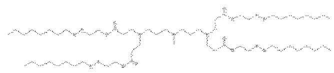 |
| L-3190 | 9-[4-(dimethylamino)-1-oxobutoxy]-heptadecanedioic acid, 1,17-di-(2Z)-2-nonen-1-yl ester | 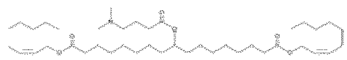 |

According to second aspect of the present invention, there is provided a vector, or more than one vector, wherein the at least one vector comprises at least one nucleic acid molecule encoding the modified RNA according to the present invention.

A vector designed for mRNA production is a DNA construct engineered to facilitate the synthesis of specific mRNA molecules. It comprises certain genetic elements, including a promoter, coding sequence, optionally a polyadenylation signal, and may incorporate other components like selection markers and origins of replication. A vector according to the present invention are based on plasmids. These plasmid backbones contain essential elements required for the replication and maintenance of the vector within the host cell.

In certain preferred embodiments according to the present invention there is provided a vector, wherein the at least one region encoding for the mRNA or a combination of mRNAs is defined by SEQ ID NO: 3 to 8 or a sequence having at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity to any of the SEQ ID NOs: 3 to 8, respectively.

According to a third aspect of the present invention there is provided a pharmaceutical formulation comprising the composition according to the present invention, wherein the pharmaceutical formulation additionally comprises at least one pharmaceutically acceptable carrier or excipient.

In certain embodiments, the pharmaceutically acceptable carrier or excipient comprises at least one bulking agent and/or at least one binder and/or at least one disintegrant and/or at least one coating and/or at least one filler or diluent and/or at least one stabilizer or preservative and/or at least one solvent and/or at least one solubilizer.

One of the primary functions of a pharmaceutically acceptable carrier or excipient is to provide the necessary bulk

and form to create various dosage forms, including suspensions, or injectable solutions, ensuring the integrity of the final product. Additionally, carriers or excipients play a pivotal role in enhancing the stability of the active pharmaceutical ingredient (API), shielding it from factors like light, heat, moisture, or chemical reactions that could lead to degradation.

A critical aspect of these carrier or excipients is their ability to adjust the pH of the formulation to levels conducive to the stability and efficacy of the API. Importantly, these substances must not interact adversely with the API or other components, ensuring the safety and integrity of the medicinal product. Pharmaceutically acceptable carriers and excipients adhere to stringent quality and safety standards, ensuring they do not introduce impurities, contaminants, or adverse effects into the pharmaceutical product. They are indispensable in drug development, allowing for the creation of formulations suitable for patient administration, ultimately delivering the desired therapeutic effect while upholding safety and stability standards.

One embodiment relates to the pharmaceutical formulation according to the previous aspect for use in a method of treating a disease associated with the formation of extracellular DNA, preferably neutrophil extracellular traps (NETs), comprising systemically or locally administering said pharmaceutical formulation to a subject, wherein the subject is a human or a non-human mammal, optionally wherein the pharmaceutical formulation is administered in combination with an immunotherapeutic agent.

An immunotherapeutic agent, provided to a patient in the form of immunotherapy, designed to manipulate the body's immune system to combat diseases, such as cancer and inflammatory diseases. Such an agent enhances the immune system's ability to detect and destroy harmful cells or pathogens in various ways. Immunotherapeutic agents comprise cancer vaccines, checkpoint inhibitors, functionalized T-cells in adoptive cell transfer and/or checkpoint inhibitors. Cancer vaccines are designed to treat cancer by stimulating the immune system to attack cancer cells. Adoptive cell transfer involves using patients' immune cells, modified and multiplied outside the body, to treat their cancer, with CAR T-cell therapy being a prominent example.

Immune checkpoints are critical regulatory pathways maintaining self-tolerance and modulating the intensity of immune responses, thereby minimizing potential damage to host tissues. Cancer cells, however, can co-opt these regulatory pathways to evade immunological detection and destruction. Programmed Death-1 (PD-1) is a prominent example of such an immune checkpoint. Expressed on T-cells, PD-1 functions as a regulatory molecule, attenuating the immune response under normal physiological conditions. It achieves this through interaction with its ligand, PD-L1, a molecule that can be expressed on various cells, including certain cancer cells. The binding of PD-1 to PD-L1 serves to inhibit T-cell activity, thereby diminishing the immune response and facilitating the survival and proliferation of cancer cells. Targeted immunotherapies that inhibit the PD-1/PD-L1 interaction have emerged as a viable strategy in the treatment of various malignancies. These immune checkpoint inhibitors, by blocking this interaction, potentiate the immune system's capacity to detect and eradicate cancer cells.

In one embodiment, the pharmaceutical formulation for use in a method of treatment according to the present invention is combined with a therapeutic antibody, preferably an immune checkpoint inhibitor (e.g. anti-PD1).

A preferable combination includes but is not limited to using the pharmaceutical compositions according to the present invention in combination with at least one therapeutic antibody in the form of recombinant protein or and/or at least one mRNA molecule encoding the therapeutic antibody.

One embodiment relates to the pharmaceutical formulation for use according to the previous embodiment, wherein the disease is selected from the group consisting of cancer, deep vein thrombosis, pulmonary embolism, disseminated intravascular coagulation, and inflammatory diseases.

This invention relates to mRNA molecules encoding recombinant DNASES and pharmaceutical compositions containing them that are suitable for gene delivery of DNASES for therapeutic purposes. This comprises the use of DNASE1 alone, DNASE1L3 alone or a combination of DNASE1 and DNASE1L3 for the treatment of cancer and other cfDNA-dependent diseases such as thrombosis and thrombosis-associated diseases, systemic lupus erythematosus (SLE), antiphospholipid syndrome, atherosclerosis, asthma, sepsis, inborn DNASES deficiencies, vasculitis, acute pancreatitis, rheumatoid arthritis, type 2 diabetes mellitus impaired wound healing, gout, inflammatory bowel disease; vascular occlusion, acute respiratory distress syndrome (ARDS), chronic obstructive pulmonary disease (COPD), acute lung injury (such as transfusion-related acute lung injury [TRALI], lung ischemia-reperfusion injury [LIRI], ventilator-induced lung injury [VILI]), periodontitis. mRNA-encoded DNASES can also be used in the prevention or management of diseases and procedures associated with the formation of microbial biofilms in the course of tracheobronchial infections or prevention and control of biofilm-based medical-device-related infections associated with the introduction of catheters (such as central venous catheters [CVCs], catheter-associated urinary tract infections [CAUTIs], implantation of orthopedic devices, intratracheal tubes, etc).

In a further aspect, there is provided a method of producing the at least one modified mRNA of the composition according to the present invention, the method comprising: (i) providing the at least one vector according to the second aspect, and introducing said at least one vector into at least one host cell, (ii) culturing said at least one host cell under conditions suitable for propagation and/or amplification of said at least one vector, (iii) allowing transcription from said at least one vector and obtaining said at least one modified RNA; and (iv) optionally: purifying said at least one modified RNA

of step (iii); and/or (v) optionally: formulating said at least one modified RNA yielding a pharmaceutical formulation according to the third aspect, (vi) optionally: storing said at least one vector of and/or said at least one modified RNA and/or the pharmaceutical formulation.

One embodiment relates to the method according to the previous aspect further comprising a step of (vii) introducing or contacting said at least one modified RNA obtained in step (iii) into or with at least one target cell in an in vitro cellular system for transfecting said at least one target cell and/or for in vitro studying the functionality of said at least one modified RNA.

One aspect of the present invention relates to a kit comprising the at least one modified mRNA of the composition, the at least one vector and/or the pharmaceutical formulation according to the present invention, optionally wherein the kit comprises at least one further reagent and/or at least one aqueous solution, preferably a buffer.

In certain embodiments, the mRNA or the pharmaceutical composition may be provided in lyophilized form or suspended in an aqueous buffer such as Tris-HCL, HEPES, PBS or Bis-Tris, but not limited thereto, wherein the buffer preferably has usuall a pH of about pH 6.5 to about 8.5.

In certain embodiments, the kit may include a first container with a solution, preferably a buffered solution for dilution of LNPs to a desired concentration prior to reconstitution of the LNP-encapsulated modified mRNA, and a second container with the mRNA or the pharmaceutical composition in lyophilised form or suspended in an aqueous buffer as defined above, and a third container with LNP forming reagents, such as lipids, in solid form or suspended in an alcohol, and, optionally, another container with a solution, preferably a buffered solution for dilution of the mRNA, the pharmaceutical composition and/or the LNPs to the desired concentrations before use.

Further, the kit may contain accessories such as membranes, tubes and filters for concentration and/or purification of LNPs. The kit may also include detailed instructions for the reconstitution of the lyophilized components, preparation of the injectable solution, and guidelines for administration to ensure safety and efficacy.

The present invention is further illustrated by the following, non-limiting examples.

## Examples

Example 1: Preparation of DNA plasmid templates for DNASE1 and DNASE1L3 genes.

Human deoxyribonuclease 1 gene (DNASE1) and human deoxyribonuclease 1L3 (DNASE1L3) genes were purchased from Invitrogen (ThermoFisher Scientific) in plasmid vectors. The proteins sequences are defined by the respective SEQ ID NOs: 1 and 2, while the respective DNA sequences are defined by SEQ ID NOs: 3 to 5 encoding for DNASE1 and SEQ ID NOs: 6 to 8 encoding for DNASE1L3. In order to obtain a DNA template with a poly(A) tail, a several-step procedure was carried out to insert an adenine oligonucleotide into the 3'end of DNASE sequence. The insert was designed as a double-stranded DNA oligonucleotide and added to plasmid vectors using a blunt end cloning method. Solutions of two DNA oligonucleotides (Genomed) with poly(A) sequence (coding strand); and poly(T) (template strand) were mixed in a 1:1 ratio and an enzymatic reaction was set up to phosphorylate the 5' ends of the oligonucleotides by adding T4 Polynucleotide Kinase (NEB) in the T4 Polynucleotide Kinase (NEB). The insert DNA was purified using a commercial DNA purification kit (Macherey-Nagel) according to the protocol. The plasmids coding each DNASE1 and DNASE1L3 variants (2 μg) were linearized with AarI restriction enzyme using the manufacturers protocol (ThermoFisher Scientific) and purified using a commercial DNA purification kit (Macherey-Nagel). The enzymatic reaction was then prepared with DNA Polymerase I, Large Fragment (Klenow), 10× buffer 3(NEB), and 10 mM NTP (Thermo Fisher Scientific) to remove 3' overhangs and filling in 5' overhangs to form blunt ends and again purified the vector using a commercial DNA purification kit (Macherey-Nagel). Finally, dephosphorylation reaction was performed using Fast AP Thermosensitive Alkaline Phosphatase (ThermoFisher Scientific) and the final vector was purified using a commercial DNA purification kit (Macherey-Nagel).The solutions of the final vectors encoding DNASE1 or DNASE1L3 containing 100 ng of DNA were mixed with the insert at a molar ratio of 1:3 (vector-insert), and incubated in the presence of ATP (ThermoFisher Scientific) and T4 DNA Ligase (ThermoFisher Scientific). The ligation mixture (10 μl) was then used to transform commercially available chemo competent Top10 bacteria (50 μl) (ThermoFisher Scientific) using heat-shock method. The transformation mixture (200 μl) was spread on LB-agar plate (Roth) with 100 mg/mL Ampicillin (Roth) and incubated (37°C, 16 h). Then, single colonies were selected and inoculated with liquid LB medium (Roth) in a volume of 5mL and supplemented with Ampicillin (Roth) at a concentration of 100 mg/mL and the cultures were incubated (37°C, 16 h). The bacterial cultures were then centrifuged and the plasmids were purified using the commercial GeneJET Plasmid Miniprep Kit (ThermoFisher Scientific). Concentration was measured using a Nanodrop 2000c spectrophotometer (ThermoFisher Scientific) and plasmid identities were confirmed using the Sanger DNA sequencing method (Genomed).

Example 2: Preparation of DNASE encoding mRNA

mRNAs encoding human DNASE1 and DNASE1L3, defined by the respective SEQ ID NOs: 3 and 4, were

generated on templates generated as described in Example 1. The in vitro transcription reaction contained 40 mM BIS-TRIS buffer pH 6.5, 5 mM ATP, CTP, UTP or UTP analog and 4 mM GTP (Thermo Fisher Scientific), 25 mM $MgCl_2$, inorganic pyrophosphatase (Thermo Fisher Scientific), 10 mM cap analog, linearized plasmid as the DNA template and T7 RNA polymerase. All components were gently mixed by pipetting and incubated at 37° C for 1-2 h. The DNA template was removed by incubation with DNASE I (Thermo Fisher Scientific) and all enzymes used were inactivated by the addition of one volume of 52 mM EDTA (Chemsolute).

The products were isolated by a multi-step purification process. The oligo dT chromatography (POROS™ Oligo(dT)$_{25}$ Affinity Resin (Thermo Fisher Scientific) was used first. The crude IVT mixes were adjusted to achieve the high salt buffer conditions and loaded at room temperature onto the accordingly equilibrated oligo(dT)$_{25}$ resin for the binding step. The resin was washed with high salt buffer and successively with low salt buffer. Elution of mRNA was conducted at 65°C with nuclease-free water (Thermo Fisher Scientific). The obtained samples were next filtrated through 0.2 $\mu$m sterile syringe filters (Alchem) and concentrated with Amicon® Ultra Centrifugal Filter Units with 100 kDa MWCO (Merck Millipore).

RP-HPLC was used to separate the capped mRNAs of interest from uncapped fractions, dsRNA and other contaminants remaining after preliminary purification. Semi-preparative HPLC was performed on an Agilent 1260 Infinity II with UV-Vis/DAD-detection at 256nm using RNASep™ Semi-Prep column (ADS Biotec). The linear gradient of acetonitrile in 0.1 M triethylammonium acetate (TEAA) pH 7.0 at 55°C was used for the elution of each mRNA. The quality of collected fractions was analyzed on 1× TBE 1.2% agarose gel. Selected fractions were combined and the eluate was desalted by ultrafiltration (Amicon Ultra-15 100K, Millipore). mRNAs were precipitated with 0.3 M sodium acetate (NaOAc) pH 5.2 with one volume of isopropanol at -20° C overnight and washed with the cold 80% ethanol. mRNA pellets were resuspended in nuclease-free water (Thermo Fisher Scientific). mRNA purity and homogeneity was confirmed by gel electrophoresis (FIG 2), dot-blot analysis (FIG 3), and HPLC (FIG 4).

Example 3: mRNA formulation

The SM-102 (cat#BP-25499) lipid was purchased from BroadPharm (USA). The 1,2-dimyristoyl-rac-glycero-3-methoxypolyethylene glycol-2000 (DMG-PEG2k, cat#880151P), cholesterol (ovine wool, cat#700000P) and 1,2-dioctadecanoyl-sn-glycero-3-phosphocholine (DSPC, cat#850365P) were purchased from Avanti Polar Lipids (USA). The stock solutions of lipids were prepared in absolute ethanol (Thermo Fisher Scientific, cat#BP2818500) at the concentration of 100 mg/mL for SM-120 and 10 mg/mL for the rest of the lipids. The lipid mix was prepared by combining SM-102, DSPC, cholesterol and DMG-PEG2k at molar ratio of 50:10:38.5:1.5 in absolute ethanol at total concentration of 15 mM or 25 mM. Stock solution of mRNA was diluted in 100 mM sodium citrate buffer pH 4.0 at the mRNA final concentration of 102 ng/$\mu$L or 170 ng/$\mu$L. The lipid mix and mRNA solution were combined in a microfluidic device (NanoAssemblr® Ignite™, Precision NanoSystems) equipped with NxGen Cartridge (cat#NIN0002) at a flow ratio of 4:1 (aqueous phase:ethanol) with a total flow rate of 12 mL/min. The final weight ratio of SM-102:mRNA was 13:1 and N/P ratio (cationic nitrogen groups from the ionizable lipid over anionic phosphate groups from the mRNA) was ~6.

All resulting LNP nanoparticles (LNPs) were diluted ~40 times in 1xPBS sterile buffer (w/o calcium and magnesium ions) and concentrated by ultrafiltration using Amicon ultracentrifugal tubes (Merck Millipore, cat# UFC9050) with 100kDa MWCO (3000 × g, 20°C). Final LNPs were stored at 4°C. Other lipid formulations were prepared analogously as described above. The compositions of representative lipid formulations are shown in Table 2.

**Table 2**

| Lipid mix | Component 1 (Ionizable cationic lipid, C1) | Component 2 (Neutral lipid, C2) | Component 3 (Sterol, C3) | Component 4 (PEG-ylated lipid, C4) | Molar Lipid ratios (%) C1:C2:C3 :C4 |
|---|---|---|---|---|---|
| Mix 1 | DLin-MC3-DMA: (6Z,9Z,28Z,31 Z)-heptatriaconta-6,9,28,31-tetra-en-19-yl-4-(dimethy-lamin o) butanoate | 1,2-Distearoyl-sn-glycero-3-phosphocholi ne (DSPC) | Cholesterol | PEG2000-DMG = Alpha-(3'-{[1,2-di(myristyloxy) propanoxy] carbonylamino } propyl)-ω-methoxy, polyoxyethylen e | 50:10:38. 5:1.5 |
| Mix 2 | ALC-0315 = (4-hy-droxybutyl) azane-diyl)bis (hexane-6,1-diyl)bis(2-hexyldecanoat e) | 1,2-Distearoyl-sn-glycero-3-phosphocholi ne (DSPC) | Cholesterol | ALC-0159 = 2-[(polyethylene glycol)-2000]-N,N ditetradecylac etamide | 46.3:9.4:4 2.7:1.6 |

(continued)

| Lipid mix | Component 1 (Ionizable cationic lipid, C1) | Component 2 (Neutral lipid, C2) | Component 3 (Sterol, C3) | Component 4 (PEG-ylated lipid, C4) | Molar Lipid ratios (%) C1:C2:C3 :C4 |
|---|---|---|---|---|---|
| Mix 3 | SM-102 (heptadecan-9-yl 8-((2-hydro-xyethyl) (6-ox-o-6-(undecyloxy) hexyl) amino) octanoate} | 1,2-Distearoyl-sn-glycero-3-phosphocholi ne (DSPC) | Cholesterol | PEG2000-DMG = 1-monomethoxy polyethylenegl ycol-2,3-dimyristylglyce rol with polyethylene glycol of average molecular weight 2000 | 50:10:38. 5:1.5 |

Size distribution and polydispersity index were determined using dynamic light scattering (DLS) on Malvern Zetasizer Ultra Red (Malvern, UK) in PBS buffer at 25°C in backscatter mode. Encapsulation efficiency and concentration of mRNA entrapped in LNPs was determined using the Quant-iT Ribogreen RNA assay (Thermo Fisher Scientific, USA) by comparing fluorescence intensities in the presence or absence of 0.1% (w/v) Triton X-100. Typical sizes of the prepared LNPs were <100 nm, polydispersity indexes <0.16, and encapsulation efficiencies >88%.

Example 4: Analysis of hDNASE1 and hDNASE1L3 activity towards NETs in cell culture medium

$0.5\times10^3$ HEK293 cells were seeded on a 6 well plate. Next day the medium was changed for 1 ml of AIMV and cells were transfected with 800 ng mRNA encoding hDNASE1, hDNASE1L3 or hEPO with 1.5 μl Lipofectamine MessengerMax. After 24 h the medium was collected and centrifuged to get rid of the remaining cells.

$100\times10^3$ of freshly isolated human peripheral blood neutrophils were seeded on 48-well plate in 200 μl HBSS. Next, 100 nM PMA was added to induce NET (genomic DNA-containing neutrophil extracellular traps) formation. After 1 h 10 U/ml Pulmozyme® or 25 μl of conditioned medium were added to the corresponding groups. After 2 h of incubation 1 μM SYTOX Green was added and the cells were observed under Nikon Eclipse TE2000 fluorescent inverted microscope at 100x magnification with QICAM 12-bit monochrome digital camera using ImageProPlus software. The results are shown in FIG 5.

Example 5: Analysis of hDNASE1, hDNASE1L3 activity towards plasmid DNA in cell culture medium

$0.5\times10^3$ HEK293 cells were seeded on 6 well plate. Next day medium was changed for 1 ml of AIMV and cells were transfected with 800 ng mRNA encoding hDNASE1, hDNASE1L3 or hEPO with 1.5 μl Lipofectamine MessengerMax After 24h medium was collected and centrifuged to get rid of the remaining cells.

320 ng of linearized plasmid DNA was incubated at 37°C for 1 h with 10 μl of HBSS with calcium and magnesium ions and 10 μl of conditioned medium from mRNA-transfected HEK293 cells. Next, samples were analyzed using 1% agarose in TE buffer electrophoresis. The results are shown in FIG 6.

Example 6: Measuring in vivo activity of pharmaceutical compositions comprising DNASE-encoding mRNA

C57BL/6 mice were inoculated with murine colon adenocarcinoma (MC38) cells and treated 3 times a week i.p. (for 2 consecutive weeks) with 10 μg of $m^7Gppp^{Bn6}A_mpG$-capped mRNA for hDNASE1, hDNASE1L3 or hEPO formulated in SM-102. Pulmozyme®-treated group received 25 U of the drug at the same schedule. mRNA for hEPO (protein without endonuclease activity) served as internal mRNA control. At day 0 of the experiment, $1\times10^6$ MC38 cells were inoculated subcutaneously into the fat pad of female 8-12-week-old C57BL/6 mice. Mice were observed for tumor appearance and tumor growth was measured with digital calipers (AOS 100 mm with roll). Tumor dimensions: maximum length and width were measured 3 times a week, and the tumor volume was calculated according to the following formula:

$$TV = \frac{1}{2} \times a^2 \times b,$$

where TV is the tumor volume, a is the shorter diameter and b is the longer diameter. To compare tumor growth-inhibiting activity between the experimental groups, tumor growth inhibition values (TGI) were estimated using the following formula:

$$TGI=100-TVN/TVC\times100,$$

where TGI is the tumor growth inhibition value, TVN is the mean tumor volume calculated for the studied experimental group and TVC is the mean tumor volume calculated for the control group of animals. Tumor volumes are shown as means $\pm$ error, n=5. Mixed-effects analysis with Dunnett's multiple comparisons test, *P<0.05 hDNASE1L3 vs control group. TGI - is tumor growth inhibition compared with controls. mRNA for hDNASE1, hDNASE1L3, hEPO (protein without endonuclease activity) or Pulmozyme® (recombinant DNASE) was administered intraperitoneally (i.p.) on days 7, 9, 11, 14, 16, 18, 21 and 23 after inoculation of tumor cells. The results are shown in FIG 7.

Example 7. Measuring DNAse activity in mouse serum after administration of DNAse encoding mRNA or Pulmozyme®.

Mice were injected intravenously (IV) with 10 or 50 microg of SM-102-formulated mRNA (obtained as described in Example 3) for human DNAse1 or Pulmozyme® (recombinant human DNAse1 protein). Blood was collected at indicated timepoints and DNAse1 activity was measured using resDetect™ DNase Activity Assay Kit (Fluorescence) from AcroBiosystem. Pulmozyme® at 10 U and 50 U doses corresponding to 10 and 50 microg of the protein, respectively, was for comparison and a standard to calculate U/ml to microg/ml concentrations. The results are shown in FIG 8.

Example 8: Measuring in vitro activity of DNASE1 in cell culture medium after transfection with differently capped mRNAs

mRNAs capped with ARCA, $m^7GpppA_mpG$ (cap-1), $m^7Gppp_{5'}{}^{S}A_mpG$, $m^7GpppBn^6A_mpG$, or $m^7GpppA_mpG_mpG$ were generated as described in Example 2. AP34 HEK293T cells were grown for 24 h at $1 \times 10^6$ cells per well in 6 well plate. Cells were transfected with 1 $\mu$g mRNA (AP34) encoding DNase 1 and incubated at 37°C, 5% $CO_2$ for 24 h. Medium was collected and analyzed using resDetect DNase Activity Assay Kit. The activity of DNASE was measured according to the protocol for samples with interference. Samples were diluted 250-fold.

**References**

1. Mattox AK, Douville C, Wang Y, et al. The Origin of Highly Elevated Cell-Free DNA in Healthy Individuals and Patients with Pancreatic, Colorectal, Lung, or Ovarian Cancer. Cancer Discov. Oct 5 2023;13(10):2166-2179. doi:10.1158/2159-8290.CD-21-1252

2. Kaltenmeier C, Yazdani HO, Morder K, Geller DA, Simmons RL, Tohme S. Neutrophil Extracellular Traps Promote T Cell Exhaustion in the Tumor Microenvironment. Front Immunol. 2021;12:785222. doi:10.3389/fimmu.2021.785222

3. He XY, Ng D, Egeblad M. Caught in a Web: Emerging Roles of Neutrophil Extracellular Traps in Cancer. Annu Rev Canc Biol. 2022;6:223-243. doi:10.1146/annurev-cancerbio-080421-015537

4. de Andrea CE, Ochoa MC, Villalba-Esparza M, et al. Heterogenous presence of neutrophil extracellular traps in human solid tumours is partially dependent on IL-8. J Pathol. Oct 2021;255(2):190-201. doi:10.1002/path.5753

5. Mousset A, Lecorgne E, Bourget I, et al. Neutrophil extracellular traps formed during chemotherapy confer treatment resistance via TGF-beta activation. Cancer Cell. Apr 10 2023;41(4):757-775 e10. doi:10.1016/j.ccell.2023.03.008

6. Shahzad MH, Feng L, Su X, et al. Neutrophil Extracellular Traps in Cancer Therapy Resistance. Cancers (Basel). Mar 7 2022;14(5)doi:10.3390/cancers14051359

7. Pressler T. Review of recombinant human deoxyribonuclease (rhDNASE) in the management of patients with cystic fibrosis. Biologics. Dec 2008;2(4):611-7. doi:10.2147/btt.s3052

8. Davis JC, Jr., Manzi S, Yarboro C, et al. Recombinant human DNASE I (rhDNASE) in patients with lupus nephritis. Lupus. 1999;8(1):68-76. doi:10.1191/096120399678847380

9. Boogaard R, Smit F, Schornagel R, et al. Recombinant human deoxyribonuclease for the treatment of acute asthma in children. Thorax. Feb 2008;63(2):141-6. doi:10.1136/thx.2007.081703

10. Chia AC, Menzies D, McKeon DJ. Nebulised DNASE post-therapeutic bronchoalveolar lavage in near fatal asthma exacerbation in an adult patient refractory to conventional treatment. BMJ Case Rep. Jun 25 2013;2013doi:10.1136/bcr-2013-009661

11. Hull JH, Castle N, Knight RK, Ho TB. Nebulised DNASE in the treatment of life threatening asthma. Resuscitation. Jul 2007;74(1):175-7. doi:10.1016/j.resuscitation.2006.11.006

12. Al-Mayouf SM, Sunker A, Abdwani R, et al. Loss-of-function variant in DNASE1L3 causes a familial form of systemic lupus erythematosus. Nat Genet. Oct 23 2011;43(12):1186-8. doi:10.1038/ng.975

13. Carbonella A, Mancano G, Gremese E, et al. An autosomal recessive DNASE1L3-related autoimmune disease with unusual clinical presentation mimicking systemic lupus erythematosus. Lupus. Jun 2017;26(7):768-772. doi:10.1177/0961203316676382

14. Ozcakar ZB, Foster J, 2nd, Diaz-Horta O, et al. DNASE1L3 mutations in hypocomplementemic urticarial vasculitis syndrome. Arthritis Rheum. Aug 2013;65(8):2183-9. doi:10.1002/art.38010

15. Mayes MD, Bossini-Castillo L, Gorlova O, et al. Immunochip analysis identifies multiple susceptibility loci for systemic sclerosis. Am J Hum Genet. Jan 2 2014;94(1):47-61. doi:10.1016/j.ajhg.2013.12.002

16. Hartl J, Serpas L, Wang Y, et al. Autoantibody-mediated impairment of DNASE1L3 activity in sporadic systemic lupus erythematosus. J Exp Med. May 3 2021;218(5)doi:10.1084/jem.20201138

17. Ueki M, Kimura-Kataoka K, Takeshita H, et al. Evaluation of all non-synonymous single nucleotide polymorphisms (SNPs) in the genes encoding human deoxyribonuclease I and I-like 3 as a functional SNP potentially implicated in autoimmunity. FEBS J. Jan 2014;281(1):376-90. doi:10.1111/febs.12608

18. Weisenburger T, von Neubeck B, Schneider A, et al. Epistatic Interactions Between Mutations of Deoxyribonuclease 1-Like 3 and the Inhibitory Fc Gamma Receptor IIB Result in Very Early and Massive Autoantibodies Against Double-Stranded DNA. Front Immunol. 2018;9:1551. doi:10.3389/fimmu.2018.01551

19. Sisirak V, Sally B, D'Agati V, et al. Digestion of Chromatin in Apoptotic Cell Microparticles Prevents Autoimmunity. Cell. Jun 30 2016;166(1):88-101. doi:10.1016/j.ce11.2016.05.034

## Claims

**1.** A composition comprising at least one modified RNA, wherein the at least one modified RNA comprises or consists of

(i) a synthetic cap structure, wherein the synthetic cap structure is selected from the group of formula (I)

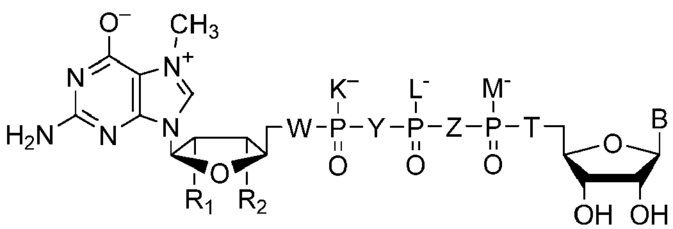

(I),

wherein B is a natural, modified, or unnatural nucleobase,

W and T are independently selected from O, S, $CH_2$, $CF_2$ and $CCl_2$,
Y, and Z are independently selected from O, $CH_2$, $CF_2$, $CCl_2$,
K, L, and M are independently selected from O, S BHs,

and $R^1$ and $R^2$ are independently selected from the group consisting of H, OH, $OCH_3$, O-alkyl, O-alkyl derivative and O-alkylaryl,

formula (II)

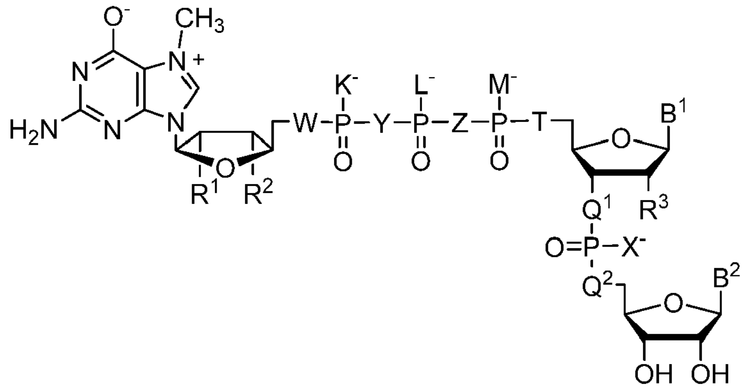

wherein $B^1$ and $B^2$ are independently natural, modified, or unnatural nucleobases,

W and T are independently selected from O, S, $CH_2$, $CF_2$, $CCl_2$,
Y and Z are independently selected from O, $CH_2$, $CF_2$, $CCl_2$,
K, L, and M are independently selected from O, S BHs,
X is selected from O, S and BHs
$Q^1$ and $Q^2$ are independently selected from O, S and $CH_2$,
$R^1$ and $R^2$ are independently selected from the group consisting of H, OH, OCH3, O-alkyl, O-alkyl derivative and O-alkylaryl,
and $R^3$ is selected from the group consisting of OH, $OCH_3$, O-alkyl, O-alkyl derivative, O-alkylaryl, F, $N_3$, $SCH_3$, $NHCH_3$ and $CH_2CH_3$, or

formula III

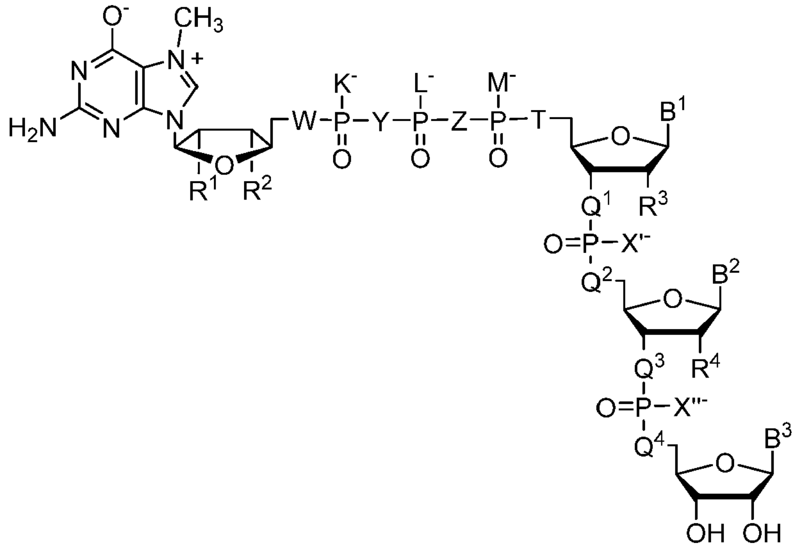

(III),

wherein $B^1$, $B^2$ and $B^3$ are independently natural, modified, or unnatural nucleobases,

W and T are independently selected from O, S, $CH_2$, $CF_2$ and $CCl_2$,
Y and Z are independently selected from O, $CH_2$, $CF_2$ and $CCl_2$,
K, L, and M are independently selected from O, S and BHs,
X' and X'' are independently selected from O, S and $BH_3$,
$Q^1$, $Q^2$, $Q^3$ and $Q^4$ are independently selected from O, S and $CH_2$,
$R^1$ and $R^2$ are independently selected from the group consisting of H, OH, $OCH_3$, O-alkyl, O-alkyl derivative and O-alkylaryl,
and $R^3$ and $R^4$ are independently selected from the group consisting of OH, $OCH_3$, O-alkyl, O-alkyl derivative, O-alkylaryl, F, $N_3$, $SCH_3$, $NHCH_3$ and $CH_2CH_3$; and

(ii) at least one modified coding region encoding at least one protein, wherein the at least one protein is a DNASE1 or a DNASE1L3, or a respective homolog, ortholog or paralog thereof, preferably encoding for both DNASE1 and DNASE1L3 or a respective homolog, ortholog or paralog thereof.

**2.** The composition according to claim 1, wherein the DNASE1 and the DNASE1L3, or a respective homolog, ortholog or paralog thereof, is individually selected from a mammalian protein, preferably from a human protein.

**3.** The composition according to claims 1 and 2, wherein the DNASE1 and/or the DNASE1L3, or a respective homolog, ortholog or paralog thereof is fused to a targeting moiety, preferably a therapeutic antibody.

**4.** The composition according to any of the preceding claims, wherein the at least one modified RNA comprises or consists of a 5'-UTR and a 3'-UTR.

**5.** The composition according to any of the preceding claims, wherein the at least one modified RNA comprises or consists of a poly-A tail, wherein the poly-A tail comprises at least

(i) 100 adenines or,
(ii) 120 adenines or,

(iii) 150 adenines or,
(iv) 250 adenines, or
(vi) a segmented poly-A tail comprising at least 150 adenines, wherein each segment is defined by the formula $A_nXA_nXA_nXA_n(XA_n)_m$, wherein A is a 5'-adenylyl moiety, X is a 5'-guanylyl or 5'-cytidylyl moiety, n is any natural number between 26 and 34, and m is 1, 2 or 3.

**6.** The composition according to any of the preceding claims, wherein the modified mRNA is a self-amplifying RNA (saRNA) and/or a circular RNA.

**7.** The composition according to any of the preceding claims, wherein the at least one modified coding region encodes for a protein or a combination of proteins defined by SEQ ID NO: 1 and 2 or a sequence having at least 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98% or 99% sequence identity to any of the SEQ ID NOs: 1 and 2 respectively,

**8.** The composition according to any of the preceding claims, wherein said composition is provided together with at least one carrier, said carrier comprising at least one lipid nanoparticle, wherein the at least one lipid nanoparticle is selected from the group of liposomes (LPs), liposome-like nanoparticles (LLPs), solid lipid nanoparticles (SLNs), nanostructured lipid carriers (NLCs), lipid-polymer hybrid nanoparticles (LPNs), lipoprotein particles (LPTs), nanoemulsions, cationic nanoemulsions (CNE) and exosomes.

**9.** A vector, or more than one vector, wherein the at least one vector comprises at least one nucleic acid molecule encoding the modified RNA of the composition according to any of the preceding claims.

**10.** A pharmaceutical formulation comprising the composition according to any of claims 1 to 8, wherein the pharmaceutical formulation additionally comprises at least one pharmaceutically acceptable carrier or excipient.

**11.** The pharmaceutical formulation according to claim 10 for use in a method of treating a disease associated with the formation of extracellular DNA, preferably neutrophil extracellular traps (NETs), comprising systemically or locally administering said pharmaceutical formulation to a subject, wherein the subject is a human or a non-human mammal, optionally wherein the pharmaceutical formulation is administered in combination with at least one immunotherapeutic agent.

**12.** The pharmaceutical formulation for use according to claim 11, wherein the disease is selected from the group consisting of cancer, deep vein thrombosis, pulmonary embolism, disseminated intravascular coagulation, and inflammatory diseases.

**13.** A method of producing the at least one modified mRNA of the composition according to any of claims 1 to 8, the method comprising:

(i) providing the at least one vector according to claim 8, and introducing said at least one vector into at least one host cell,
(ii) culturing said at least one host cell under conditions suitable for propagation and/or amplification of said at least one vector,
(iii) allowing transcription from said at least one vector and obtaining said at least one modified RNA; and
(iv) optionally: purifying said at least one modified RNA of step (iii); and/or
(v) optionally: formulating said at least one modified RNA yielding a pharmaceutical formulation according to claim 9,
(vi) optionally: storing said at least one vector of and/or said at least one modified RNA and/or the pharmaceutical formulation.

**14.** The method of claim 13 further comprising a step of
(vii) introducing or contacting said at least one modified RNA obtained in step (iii) into or with at least one target cell in an in vitro cellular system for transfecting said at least one target cell and/or for in vitro studying the functionality of said at least one modified RNA.

**15.** A kit comprising the at least one modified mRNA of the composition as defined in claims 1 to 8, the at least one vector according to claim 9 and/orthe pharmaceutical formulation according to claims 10 to 12, optionally wherein the kit comprises at least one further reagent and/or at least one aqueous solution, preferably a buffer.

FIG 1

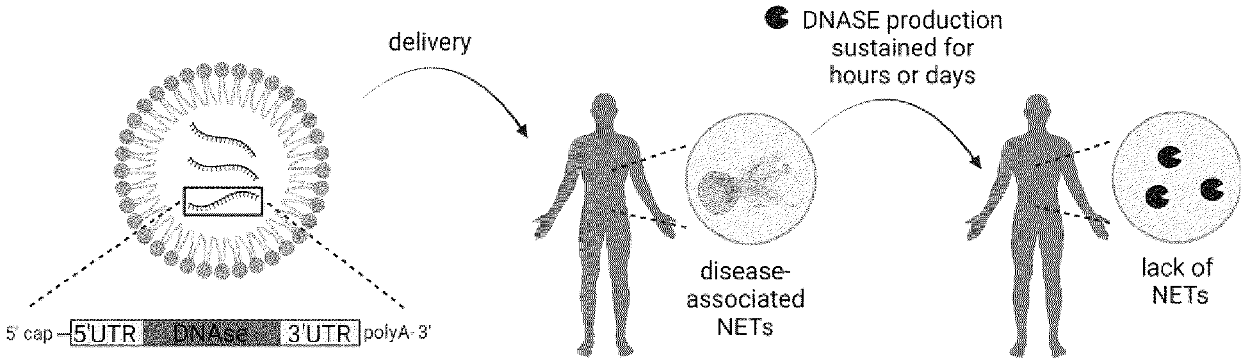
delivery
DNASE production
sustained for
hours or days
5' cap
5'UTR
DNAse
3'UTR
polyA- 3'
disease-
associated
NETs
lack of
NETs

FIG 2

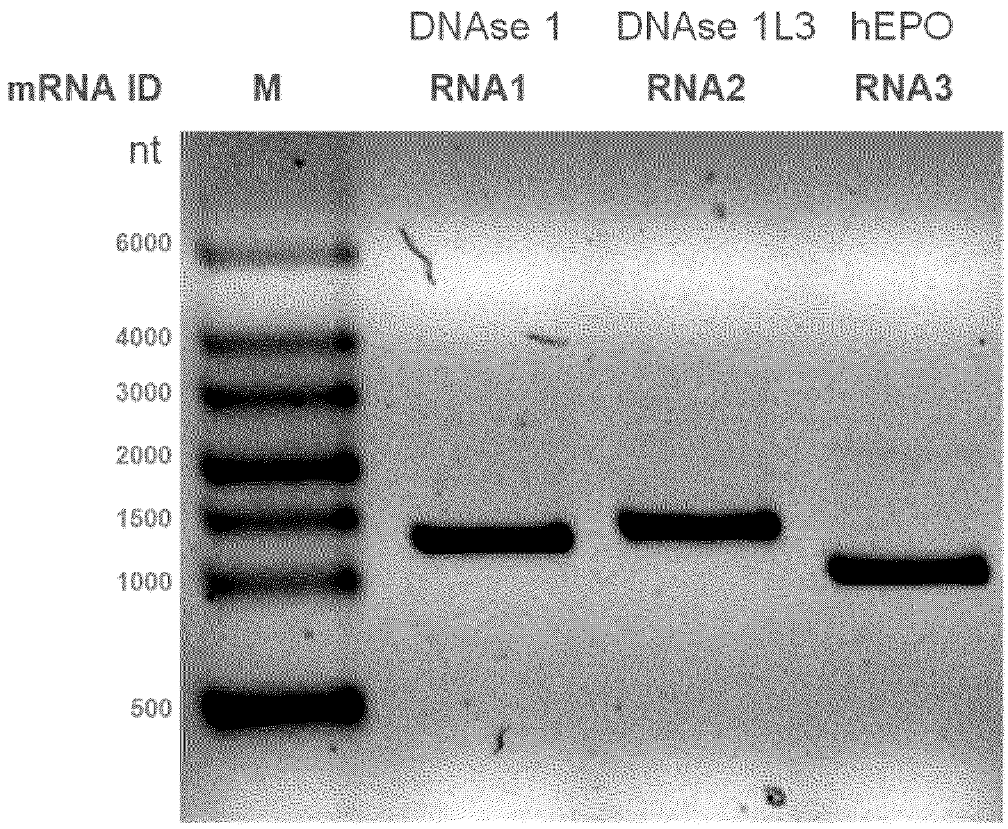
DNAse 1
DNAse 1L3
hEPO
mRNA ID
M
RNA1
RNA2
RNA3
nt
6000
4000
3000
2000
1500
1000
500

FIG 3

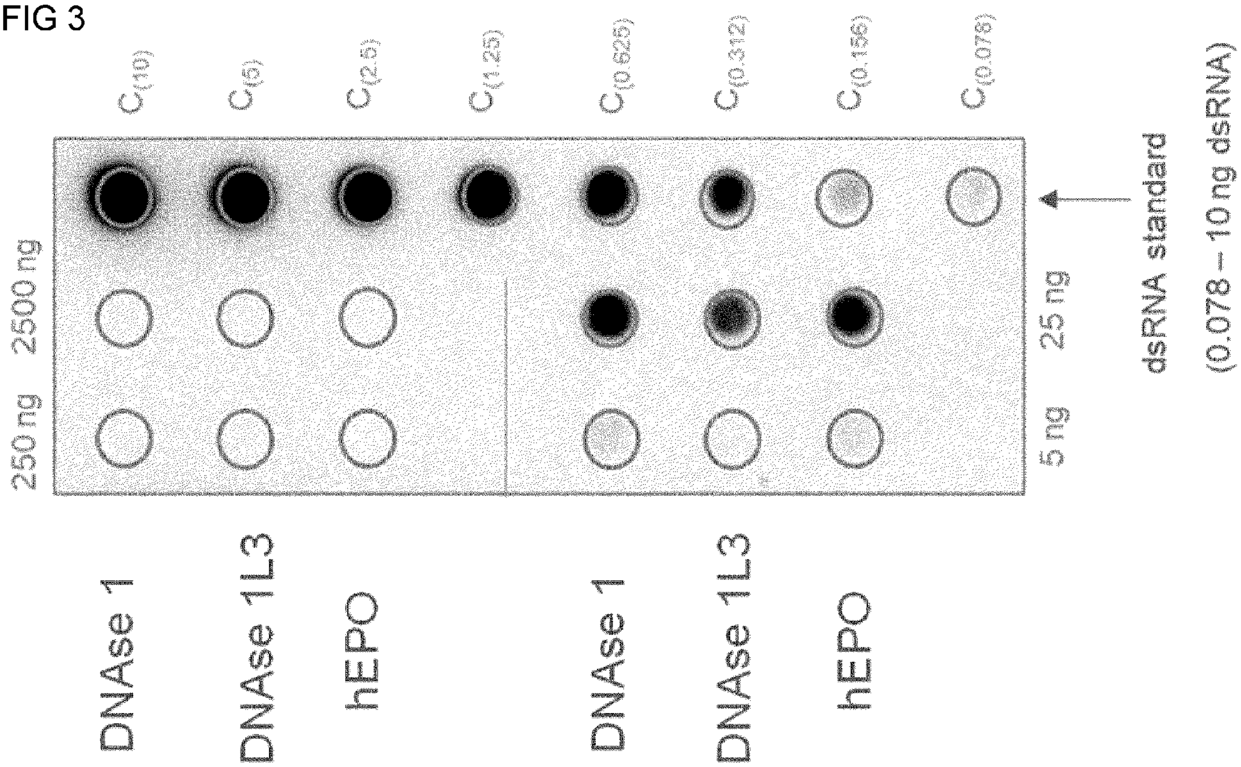
$C_{(10)}$
$C_{(5)}$
$C_{(2.5)}$
$C_{(1.25)}$
$C_{(0.625)}$
$C_{(0.312)}$
$C_{(0.156)}$
$C_{(0.078)}$
2500 ng
250 ng
25 ng
5 ng
dsRNA standard
(0.078 – 10 ng dsRNA)
DNAse 1
DNAse 1L3
hEPO
DNAse 1
DNAse 1L3
hEPO

FIG 4

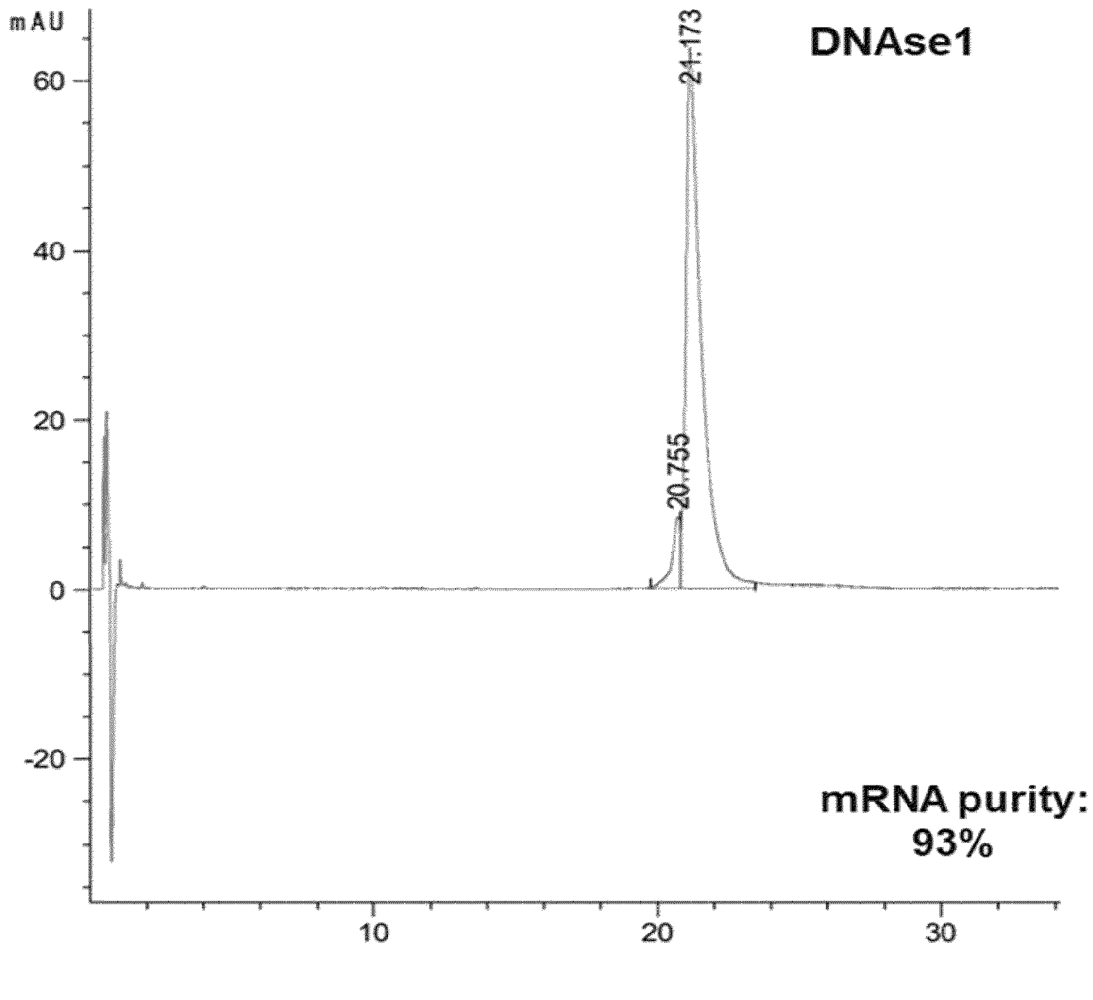

FIG 5

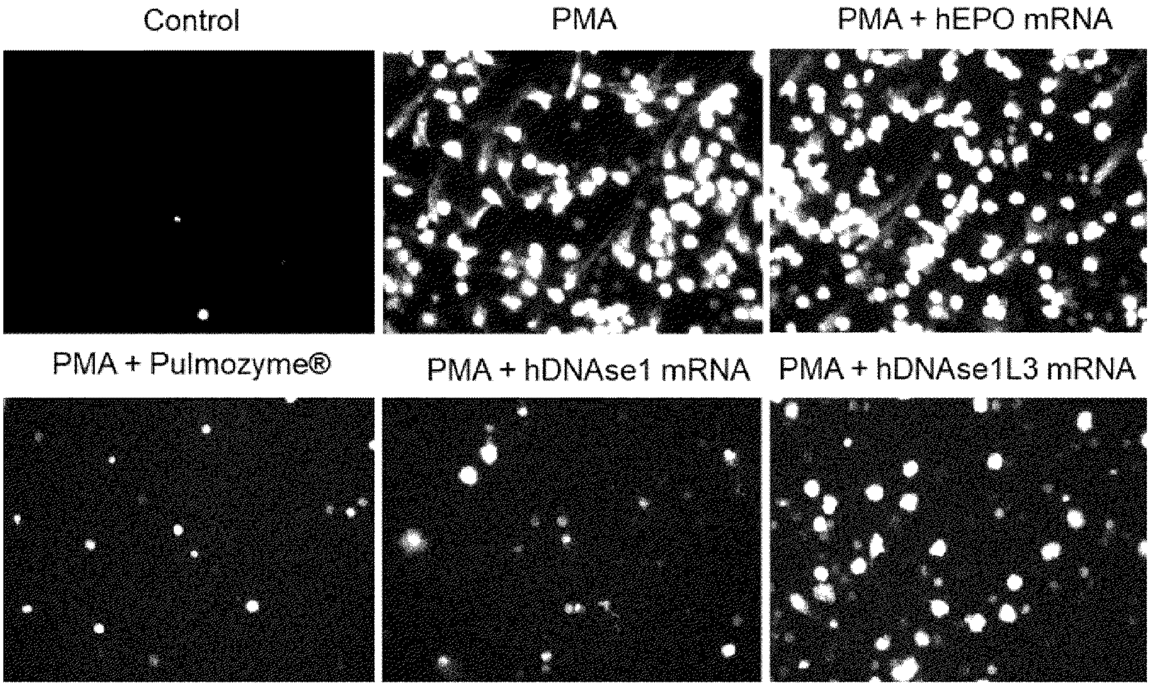
Control
PMA
PMA + hEPO mRNA
PMA + Pulmozyme®
PMA + hDNAse1 mRNA
PMA + hDNAse1L3 mRNA

FIG 6

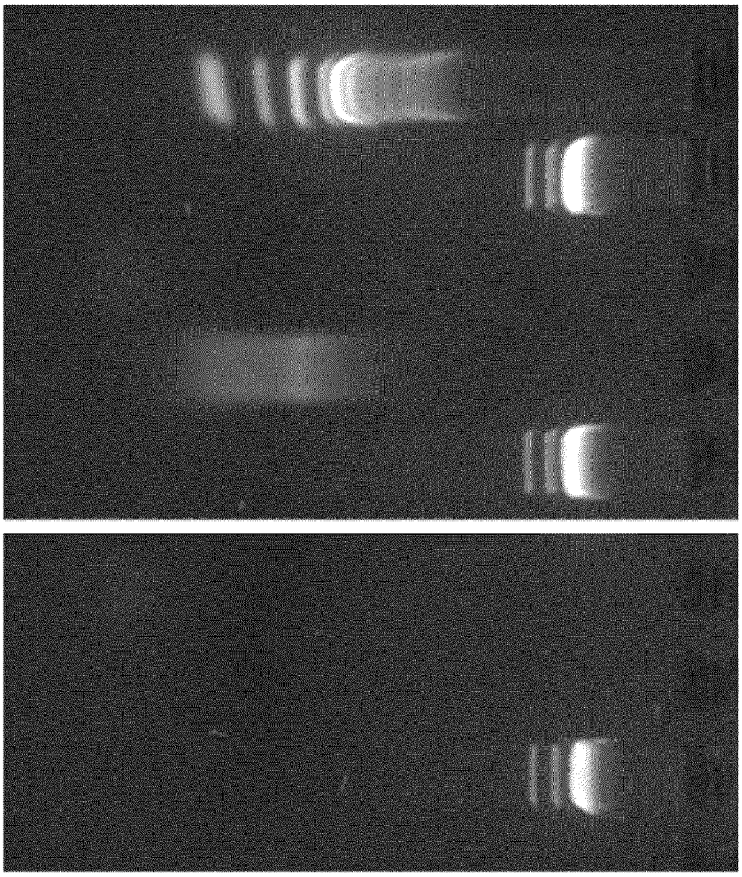

DNA size marker

Control

hDNAse1 mRNA

hDNAse1L3 mRNA

hEPO mRNA

Pulmozyme® 1U/ml

Pulmozyme® 5U/ml

Control

FIG 7

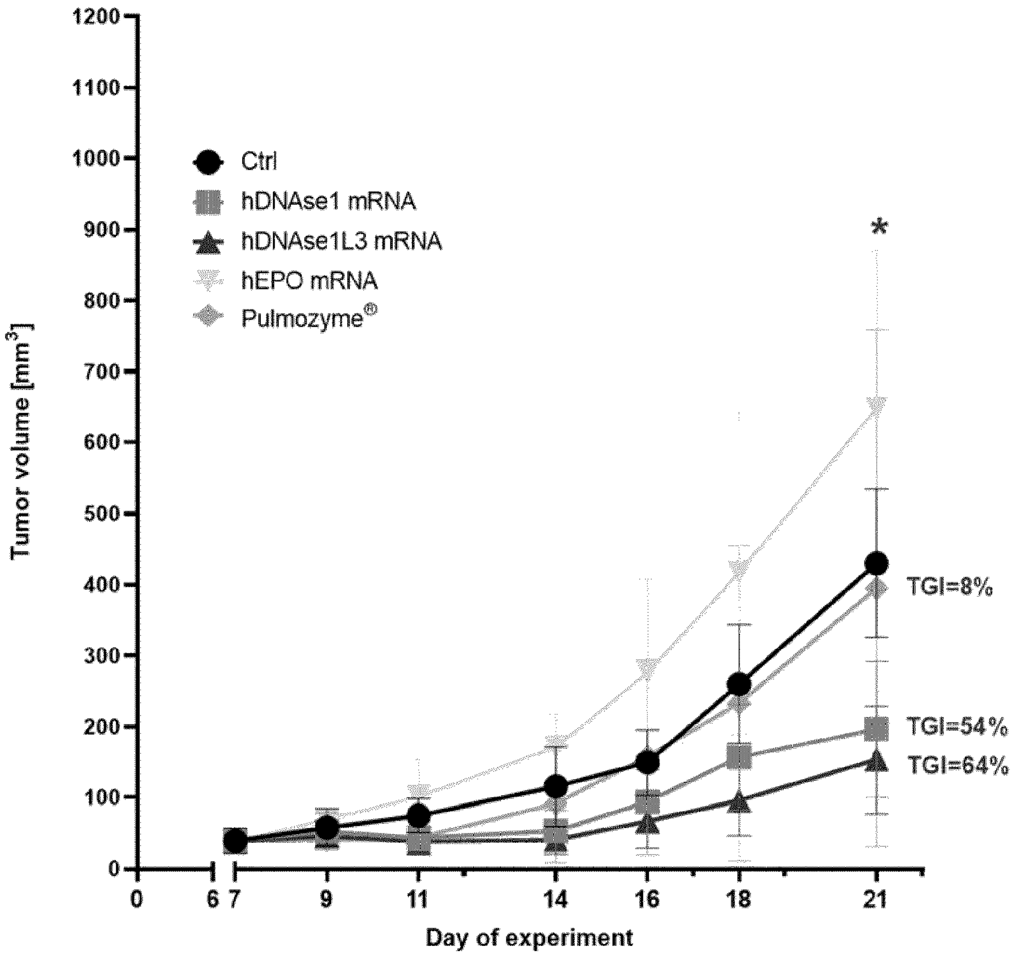
Tumor volume [mm3]
1200
1100
1000
900
800
700
600
500
400
300
200
100
0
Ctrl
hDNAse1 mRNA
hDNAse1L3 mRNA
hEPO mRNA
Pulmozyme®
*
TGI=8%
TGI=54%
TGI=64%
0
6
7
9
11
14
16
18
21
Day of experiment

FIG 8

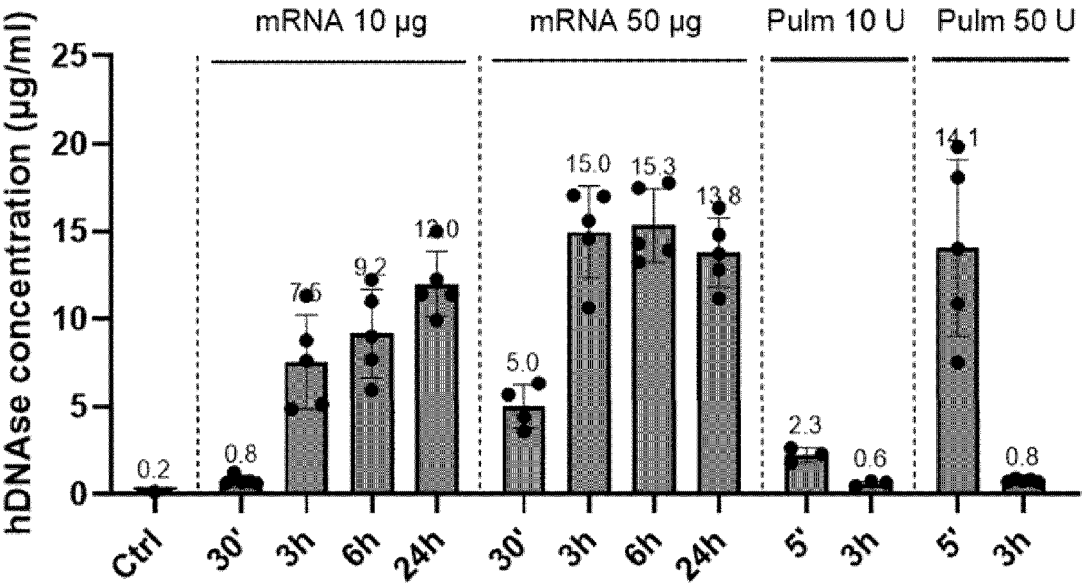

mRNA 10 µg
mRNA 50 µg
Pulm 10 U
Pulm 50 U
hDNAse concentration (µg/ml)
25
20
15
10
5
0
0.2
0.8
7.6
9.2
12.0
5.0
15.0
15.3
13.8
2.3
0.6
14.1
0.8
Ctrl
30'
3h
6h
24h
30'
3h
6h
24h
5'
3h
5'
3h

Europäisches Patentamt
European Patent Office
Office européen des brevets

# EUROPEAN SEARCH REPORT

Application Number
EP 24 46 1529

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br>Y | CN 116 463 366 A (UNIV NANKAI)<br>21 July 2023 (2023-07-21)<br>* examples 2,3,5 * | 11,12<br><br>8,11,12 | INV.<br>C12N15/85<br>C12N15/88 |
| Y | WO 2021/162567 A1 (UNIV WARSZAWSKI [PL]; EXPLORNA THERAPEUTICS SP Z O O [PL])<br>19 August 2021 (2021-08-19)<br>* claim 2; example 8 * | 11,12 | |
| X<br><br>Y | WO 2021/211848 A1 (CARTESIAN THERAPEUTICS INC [US]) 21 October 2021 (2021-10-21)<br>* example 11 * | 1-7,9,<br>10,13-15<br>8 | |
| A,D | SISIRAK VANJA ET AL: "Digestion of Chromatin in Apoptotic Cell Microparticles Prevents Autoimmunity",<br>CELL, ELSEVIER, AMSTERDAM NL,<br>vol. 166, no. 1, 9 June 2016 (2016-06-09), pages 88-101, XP029627879,<br>ISSN: 0092-8674, DOI:<br>10.1016/J.CELL.2016.05.034<br>* the whole document * | 1-15 | |
| | | | TECHNICAL FIELDS SEARCHED (IPC) |
| | | | C12N |

The present search report has been drawn up for all claims

1

| Place of search | Date of completion of the search | Examiner |
|---|---|---|
| Munich | 23 July 2024 | Mauhin, Viviane |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
..........................................................................
& : member of the same patent family, corresponding document

EPO FORM 1503 03.82 (P04C01)

# ANNEX TO THE EUROPEAN SEARCH REPORT ON EUROPEAN PATENT APPLICATION NO. EP 24 46 1529

This annex lists the patent family members relating to the patent documents cited in the above-mentioned European search report. The members are as contained in the European Patent Office EDP file on The European Patent Office is in no way liable for these particulars which are merely given for the purpose of information.

23-07-2024

| Patent document cited in search report | Publication date | Patent family member(s) | Publication date |
|---|---|---|---|
| CN 116463366 A | 21-07-2023 | NONE | |
| WO 2021162567 A1 | 19-08-2021 | AU 2021219563 A1 | 08-09-2022 |
| | | CA 3167570 A1 | 19-08-2021 |
| | | EP 4103577 A1 | 21-12-2022 |
| | | JP 2023513764 A | 03-04-2023 |
| | | KR 20220163359 A | 09-12-2022 |
| | | US 2023130423 A1 | 27-04-2023 |
| | | WO 2021162567 A1 | 19-08-2021 |
| WO 2021211848 A1 | 21-10-2021 | EP 4135739 A1 | 22-02-2023 |
| | | US 2023174944 A1 | 08-06-2023 |
| | | WO 2021211848 A1 | 21-10-2021 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **SMITH, T.F.** ; **WATERMAN, M.S.** Identification of common molecular subsequences. *Journal of Molecular Biology*, 1981, vol. 147 (1), 195-197
- **MATTOX AK** ; **DOUVILLE C** ; **WANG Y et al.** The Origin of Highly Elevated Cell-Free DNA in Healthy Individuals and Patients with Pancreatic, Colorectal, Lung, or Ovarian Cancer.. *Cancer Discov.*, 05 October 2023, vol. 13 (10), 2166-2179
- **KALTENMEIER C** ; **YAZDANI HO** ; **MORDER K** ; **GELLER DA** ; **SIMMONS RL** ; **TOHME S.** Neutrophil Extracellular Traps Promote T Cell Exhaustion in the Tumor Microenvironment.. *Front Immunol.*, 2021, vol. 12, 785222
- **HE XY** ; **NG D** ; **EGEBLAD M.** Caught in a Web: Emerging Roles of Neutrophil Extracellular Traps in Cancer. *Annu Rev Canc Biol.*, 2022, vol. 6, 223-243
- **DE ANDREA CE** ; **OCHOA MC** ; **VILLALBA-ESPARZA M et al.** Heterogenous presence of neutrophil extracellular traps in human solid tumours is partially dependent on IL-8. *J Pathol.*, October 2021, vol. 255 (2), 190-201
- **MOUSSET A** ; **LECORGNE E** ; **BOURGET I et al.** Neutrophil extracellular traps formed during chemotherapy confer treatment resistance via TGF-beta activation.. *Cancer Cell*, 10 April 2023, vol. 41 (4), 757-775
- **SHAHZAD MH** ; **FENG L** ; **SU X et al.** Neutrophil Extracellular Traps in Cancer Therapy Resistance. *Cancers (Basel).*, 07 March 2022, vol. 14 (5)
- **PRESSLER T.** Review of recombinant human deoxyribonuclease (rhDNASE) in the management of patients with cystic fibrosis. *Biologics*, December 2008, vol. 2 (4), 611-7
- **DAVIS JC, JR.** ; **MANZI S** ; **YARBORO C et al.** Recombinant human DNASE I (rhDNASE) in patients with lupus nephritis. *Lupus.*, 1999, vol. 8 (1), 68-76
- **BOOGAARD R** ; **SMIT F** ; **SCHORNAGEL R et al.** Recombinant human deoxyribonuclease for the treatment of acute asthma in children.. *Thorax*, February 2008, vol. 63 (2), 141-6
- **CHIA AC** ; **MENZIES D** ; **MCKEON DJ.** Nebulised DNASE post-therapeutic bronchoalveolar lavage in near fatal asthma exacerbation in an adult patient refractory to conventional treatment.. *BMJ Case Rep*, 25 June 2013
- **HULL JH** ; **CASTLE N** ; **KNIGHT RK** ; **HO TB**. Nebulised DNASE in the treatment of life threatening asthma.. *Resuscitation.*, July 2007, vol. 74 (1), 175-7
- **AL-MAYOUF SM** ; **SUNKER A** ; **ABDWANI R et al.** Loss-of-function variant in DNASE1L3 causes a familial form of systemic lupus erythematosus.. *Nat Genet.*, 23 October 2011, vol. 43 (12), 1186-8
- **CARBONELLA A** ; **MANCANO G** ; **GREMESE E et al.** An autosomal recessive DNASE1L3-related autoimmune disease with unusual clinical presentation mimicking systemic lupus erythematosus. *Lupus.*, June 2017, vol. 26 (7), 768-772
- **OZCAKAR ZB** ; **FOSTER J, 2ND** ; **DIAZ-HORTA O et al.** DNASE1L3 mutations in hypocomplementemic urticarial vasculitis syndrome.. *Arthritis Rheum*, August 2013, vol. 65 (8), 2183-9
- **MAYES MD** ; **BOSSINI-CASTILLO L** ; **GORLOVA O et al.** Immunochip analysis identifies multiple susceptibility loci for systemic sclerosis.. *Am J Hum Genet.*, 02 January 2014, vol. 94 (1), 47-61
- **HARTL J** ; **SERPAS L** ; **WANG Y et al.** Autoantibody-mediated impairment of DNASE1L3 activity in sporadic systemic lupus erythematosus. *J Exp Med.*, 03 May 2021, vol. 218 (5)
- **UEKI M** ; **KIMURA-KATAOKA K** ; **TAKESHITA H et al.** Evaluation of all non-synonymous single nucleotide polymorphisms (SNPs) in the genes encoding human deoxyribonuclease I and I-like 3 as a functional SNP potentially implicated in autoimmunity. *FEBS J.*, January 2014, vol. 281 (1), 376-90
- **WEISENBURGER T** ; **VON NEUBECK B** ; **SCHNEIDER A et al.** Epistatic Interactions Between Mutations of Deoxyribonuclease 1-Like 3 and the Inhibitory Fc Gamma Receptor IIB Result in Very Early and Massive Autoantibodies Against Double-Stranded DNA.. *Front Immunol.*, 2018, vol. 9, 1551
- **SISIRAK V** ; **SALLY B** ; **D'AGATI V et al.** Digestion of Chromatin in Apoptotic Cell Microparticles Prevents Autoimmunity.. *Cell*, 30 June 2016, vol. 166 (1), 88-101